# EUROPEAN PATENT SPECIFICATION

(11) **EP 2 258 833 B1**
(45) Date of publication and mention of the grant of the patent: **12.03.2014**
(21) Application number: 10164074.6
(22) Date of filing: 27.05.2010
(51) Int. Cl.: C12N 5/074

(54) **Isolation of a new cardiac stem cell population**
Isolierung einer neuen Herzstammzellenpopulation
Isolation d'une nouvelle population de cellules souches cardiaques

(30) Priority: 29.05.2009 EP 09161504
(43) Date of publication of application: 08.12.2010
(73) Proprietor: Virga Jesse Hospital, 3500 Hasselt (BE)
(72) Inventor: Hendrikx, Mark, 3140 Keerbergen (BE); Rummens, Jean-Luc, 3512 Hasselt (BE); Hensen, Karen, 3511 Hasselt (BE); Koninckx, Remco, 3510 Kermt (BE)
(74) Representative: Paemen, Liesbet R.J.

(56) References cited:
- HENSEN K ET AL L: "THE HUMAN HEART CONTAINS A CD34+NON-HAEMATOPOIETIC STEM CELL POPULATION", EXPERIMENTAL HEMATOLOGY (NEW YORK), vol. 37, no. 9, Suppl. 1, September 2009 (2009-09), page S57, XP007911938, & 38TH ANNUAL SCIENTIFIC MEETING OF THE ISEH-SOCIETY-FOR-HEMATOLOGY-AND STEM-CELLS; ATHENS, GREECE; SEPTEMBER 09 -12, 2009 ISSN: 0301-472X
- SPICHER ALBERT ET AL: "Phenotypic characterization of murine and human cardiac-resident progenitor cells isolated on basis of aldehyde-dehydragenase activity", CIRCULATION, vol. 116, no. 16, Suppl. S, October 2007 (2007-10), pages 167-168, XP007911930, & 80TH ANNUAL SCIENTIFIC SESSION OF THE AMERICAN-HEART-ASSOCIATION; ORLANDO, FL, USA; NOVEMBER 04 -07, 2007 ISSN: 0009-7322
- PESCHLE CESARE ET AL: "Stem cells for cardiomyocyte regeneration: state of the art.", ANNALS OF THE NEW YORK ACADEMY OF SCIENCES JUN 2005, vol. 1047, June 2005 (2005-06), pages 376-385, XP007911937, ISSN: 0077-8923
- OH H ET AL: "Cardiac progenitor cells from adult myocardium: homing, differentiatin, and fusion after infarction", PROCEEDINGS OF THE NATIONAL ACADEMY OF SCIENCES OF THE UNITED STATES (PNAS), NATIONAL ACADEMY OF SCIENCE, US, vol. 100, no. 21, 14 October 2003 (2003-10-14), pages 12313-12318, XP002984517, ISSN: 0027-8424, DOI: DOI:10.1073/PNAS.2132126100
- BARILE LUCIO ET AL: "Endogenous cardiac stem cells", PROGRESS IN CARDIOVASCULAR DISEASES, SAUNDERS, PHILADELPHIA, PA, US, vol. 50, no. 1, 1 July 2007 (2007-07-01), pages 31-48, XP002614837, ISSN: 0033-0620, DOI: DOI:10.1016/J.PCAD.2007.03.005
- WIRTHLIN ET AL: "Human Stem Cells for Tissue Repair", BIOLOGY OF BLOOD AND MARROW TRANSPLANTATION, KLUGE CARDEN JENNINGS PUBLISHING, CHARLOTTESVILLE, VA, US, vol. 14, no. 1, 4 January 2008 (2008-01-04), pages 151-153, XP022401193, ISSN: 1083-8791, DOI: DOI:10.1016/J.BBMT.2007.11.011
- LAUGWITZ KARL-LUDWIG ET AL: "Postnatal isl1+ cardioblasts enter fully differentiated cardiomyocyte lineages", NATURE, NATURE PUBLISHING GROUP, LONDON, UK, vol. 433, no. 7026, 10 February 2005 (2005-02-10), pages 647-653, XP002477756, ISSN: 0028-0836

## Description

### FIELD OF THE INVENTION

The present invention relates to a cardiac stem cel population which is obtainable from heart tissue and the use thereof in heart repair.

### BACKGROUND

For several decades the paradigm of the heart being a terminally differentiated organ was accepted. Now there is a growing body of evidence that challenges this dogma (Anversa et al. 1998, Beltrami et al. 2001). Not only is proof of dividing cardiomyocytes (Kajstura et al. 1998, Beltrami et al. 2001), it has also been demonstrated that the heart itself contains a pool of stem cells that are not mobilized bone marrow cells but actual stem cells residing in the heart (Beltrami et al. 2003). Over the years different isolation methods have been described to obtain these cardiac stem cells (CSCs) (Hierlihy et al. 2002, Bearzi et al. 2007; Goumans et al. 2007; Smith et al. 2007). Several reports describe that CSCs can be isolated based on the expression of membrane antigens like c-kit, Sca-1 or Islet-1 (Bearzi et al. 2007; Goumans et al. 2007, Laugwitz et al. 2005). Most of these isolation methods are based on the antigen-antibody interaction and magnetic bead sorting. This interaction is susceptible to non-specific binding caused by the Fc part of the antibodies. This implies that a heterogeneous population of cells could be isolated. Furthermore, markers like Sca-1 and c-kit are not only expressed by these CSCs, they are also present on the membrane of differentiating cells or other stem cell types like hematopoietic stem cells (HSCs). Other purification techniques such as the use of Hoechst 33342 or Rhodamine 123 are based on the functional properties of stem cells (Hierlihy et al. 2002, Wolf et al. 1993). This efflux of the dye is a biological process driven by the ATP-binding (ABC) cassette transporter proteins. However, populations isolated based on this biological process are often very heterogeneous (Challen et al. 2006). Therefore other isolation methods based on biological processes inside the cell are necessary to obtain a more homogeneous CSC population.

Several groups have isolated a population referred to as the cardiac side population (CSP) (Yoon et al. 2007; Pfister et al. 2005; Yamahara et al. 2008). Phenotypic characterization of CSP shows that these cells express CD31 and Sca-1 and are c-kit^{low}, CD34^{low} and CD45^{low} (Yoon et al., 2007). The lack of expression of CD34 and CD45 implies that these cells have a non-hematopoietic phenotype.

It has been described that different types of bone marrow-derived stem cells can be isolated based on their aldehyde dehydrogenase (ALDH) activity (Storms et al. 1999; Hess et al. 2004; Gentry et al., 2007).

Spicher et al. (2007) discloses a method for identifying a population of ALDH positive murine cardiac cells. No functional characterization of these cells is provided.

### SUMMARY OF THE INVENTION

The present study describes the isolation of a new population of cardiac stem cells out of heart tissue, more particularly, right atrial appendages. This population of cells is referred to with the acronym CASC herein. Both flow cytometric characterization as well as functional analysis show that these cells are true cardiac stem cells residing in the heart and not mobilized bone marrow stem cells. These cells are able to differentiate in to functional cardiomyocytes as shown by RT-PCR, immunofluorescence, TEM and electrophysiological measurements.

The novel cardiac stem cells of the invention express high levels of ALDH activity (ALDH+) and co-express CD34 on their membrane but are negative for the panhematopoietic marker CD45. Comparison of the antigen expression profile of bone marrow-derived ALDH+ cells and peripheral blood ALDH+ cells, CASCs show clearly a difference in expression of CD45 and also CD73 with bone marrow cells and peripheral blood ALDH+ cells. Also a functional comparison between bone marrow-derived ALDH+ cells and the cell population of the invention demonstrated that both cell types differ in their potential to differentiate into adipocytes, osteocytes or to generate hematopoietic colonies. Furthermore, it is demonstrated that, under the *in vitro* co-culture conditions described herein, both bone marrow-derived MSCs and HSCs do not differentiate into functional cardiomyocytes.

One aspect of the invention accordingly relates to a population of cardiac stem cells, referred to as CASC herein, characterized by the following features: the cells are positive for aldehyde dehydrogenase activity (ALDH+), positive for expression of CD34 (CD34(+)), negative for expression of c-kit (c-kit(-)) and negative for expression of CD45 (CD45(-)). They are also further characterized by expression of CD29, CD55 and CD73. These cells are further characterized by the ability to differentiate into cardiomyocytes.

Upon expansion (through cultivation ex *vivo* or *in vitro*), this population of cells maintains the aldehyde dehydrogenase activity, but looses expression of CD34. This population also maintains the ability to differentiate into cardiomyocytes. Accordingly, a further aspect of the invention relates to a population of expanded cardiac stem cells obtainable by expansion of the CASC population described herein, which expanded CASC population is positive for aldehyde dehydrogenase activity, does not express CD34, but expresses CD10 and CD13.

A further aspect of the invention provides compositions comprising the CASC population and/or expanded CASC population described herein. Such compositions may comprise other cell types. More particularly, such compositions further comprise one or more cell types selected from the group consisting of ALDH+/CD34(-) cells, c-kit positive cardiac stem cells, hematopoietic stem cells (HSCs), mesenchymal stem cells (MSCs). Particular embodiments of the invention relate to compositions comprising 80% ALDH+/CD34(-) cells and 20% ALDH+/CD34(-) cells, corresponding to the total ALDH+ population obtainable from heart tissue.

A further aspect of the invention relates to the therapeutic use of the CASCs, expanded CASCs and compositions of the present invention. Indeed, in view of their ability to differentiate into functional myocytes, these cells or cell populations and compositions have important therapeutic value in the restoration of damaged heart tissue. Thus this aspect encompasses methods of treating a patient in need thereof with a population of CASCs, expanded CASCs and/or compositions of the invention. The cell populations and compositions of the invention are envisaged for use as a medicament, more particularly for use in the treatment of heart disease and/or injury. Accordingly, the invention envisages pharmaceutical compositions comprising the CASCs, expanded CASCs of the invention. According to particular embodiments such pharmaceutical compositions are injectable solutions.

A further aspect of the invention provides scaffolds comprising the CASCs, expanded CASCs and/or compositions of the invention. Such scaffolds are typically used in the repair of serious heart damage.

A further aspect of the invention provides methods of obtaining compositions comprising the cardiac stem cells referred to herein as CASCs, which methods comprise selecting aldehyde dehydrogenase positive cells from isolated heart tissue. Such methods generate cell populations which comprise at least 70% cells which can be characterized as CASCs according to the invention, i.e. ALDH+, CD34(+), CD45(-), ckit(-) and capable of differentiating into cardiomyocytes.

The invention further provides methods of obtaining a population of cardiac stem cells from isolated heart tissue, which methods comprise selecting cells which are CD34 positive from the isolated heart tissue. In particular embodiments these methods comprise the step of selecting cells which are aldehyde dehydrogenase positive and which are CD34 positive. In further particular embodiments, these methods further comprise selecting cells which are CD45 negative. In particular embodiments of these methods, selection of aldehyde dehydrogenase positive cells comprises contacting cells isolated from heart tissue with a detectable substrate of aldehyde dehydrogenase, detecting the presence of a detectable product of aldehyde dehydrogenase and selecting the cells based on the detection of the product.

The invention further provides tools and kits suitable for the detection and/or selection of the CASCs, expanded CASCs and compositions of the invention. The invention further relates to the use of one or more markers selected from aldehyde dehydrogenase activity, CD34 and c-kit for the identification and/or isolation of cardiac stem cells or compositions comprising cardiac stem cells from heart, wherein aldehyde dehydrogenase and CD34 are positive markers and c-kit is a negative marker.

### DETAILED DESCRIPTION

The present invention will be described with respect to particular embodiments and with reference to certain Figures but the invention is not limited thereto but only by the claims. Any reference signs in the claims shall not be construed as limiting the scope.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The terms or definitions used herein are provided solely to aid in the understanding of the invention.

The present invention is based on the observation that heart tissue comprises a particular population of cardiac stem cells, which when freshly isolated are characterized by one or more, more particularly at least two, most particularly all of the following features
- Positive for aldehyde dehydrogenase activity (ALDH+)
- expression of CD34 (CD34+)
- absence of expression of c-kit (c-kit(-))
- absence of expression of CD45 (CD45(-)), and
- expression of CD29, CD55 and CD73 (CD29+, CD55+ and CD73+, respectively)

More particularly the cardiac stem cell population of the invention is characterized by aldehyde dehydrogenase activity (also referred to herein ALDH+) and expression of CD34. The cardiac stem cells are referred to with the acronym "CASCs" referring to the heart tissue in which they have first been identified (atrial appendage). CASCs are a type of CSCs also characterized by the absence of expression of c-kit which differentiates this cardiac stem cell population from the cardiac stem cells which have been previously identified in heart tissue by (Beltrami Cell 2003, Bearzi PNAS 2003). Furthermore the absence of expression of c-kit and CD45 differentiate the CASC cardiac stem cell population of the invention from hematopoietic stem cells.

The population of cardiac stem cells according to the present invention has been further characterized, in that it is capable of differentiating into myocytes which not only have marker genes but also the typical electrophysiological characteristics of cardiomyocytes. This ensures the therapeutic potential of this cell population.

The term "isolated" when referring to cells or a cell population as used herein refers to cells or a cell population collected from a mammal. Typically the collection is ensured based on detection of a cell marker, such as but not limited to an antibody.

The term "cell surface marker" is intended a protein expressed on the surface of a cell, which is detectable via specific antibodies. Typical cell surface markers that are useful in the invention include, but are not limited to, the CD (clusters of differentiation) antigens CD29, CD34, CD45, CD55, CD73. However other cellular markers described herein or known to the skilled person are equally useful.

The term "intracellular marker" as used herein refers to a gene or intracellular gene product such as an enzyme that is detectable. Examples of intracellular markers include but are not limited to aldehyde dehydrogenase (ALDH).

The terms "expression of [marker X]" as used herein when referring to a cell indicates that the cell expresses the marker at a level which is sufficient for detection, using standard detection methods. Expression of a marker is also referred to as "positively expressing", "+" or "pos". The terms "not expressing [marker X]" as used herein when referring to a cell indicates that the cell does not express the marker at a level which is sufficient for detection, using standard detection methods. Absence of expression of a marker is also referred to as "negative expression", "-", "neg".

For some markers, such as ALDH, expression or absence of expression is often in fact based on comparison with other cells which also express the marker. For these markers determining positive or negative expression is based on a threshold. Methods for determining positive or negative expression based on thresholds are known to the person skilled in the art and typically involve calibrating based on a "negative control". Accordingly, it will be understood that for these markers, reference to positive expression in fact implies "elevated expression compared to negative controls" and "negative expression" in fact refers to "reduced expression compared to positive controls".

When referring to a cell population, reference is made to a population which "expresses [marker X]" where at least 10%, 20%, or 30% or 40%, 50%, or 60% or 70%, 80%, or 90% or 95%, 96%, 97%, 98%, 99%, or even 100% of the cells within the population express the cell markers of interest. By "substantially free" is intended less than about 5%, 4%, 3%, 2%, 1 %, or even 0% of the cells in the population express the marker of interest.

The CASC cardiac stem cell population of the invention is morphologically characterized as a population of mononuclear cells with a round to oval nucleus, a basophilic cytoplasm without granulation and with a small nucleus to cytoplasm ratio.

The CASC cardiac stem cell population of the invention is further characterized by the ability to differentiate into cardiomyocytes *in vitro* or *in vivo.*

The CASC cardiac stem cell population of the present invention thus is characterized by different features, which distinguish the cell population from known stem cell populations, more particularly from hematopoietic stem cells and from the c-kit positive stem cells previously identified in heart.

More particularly, as described above, the CASC cardiac stem cell population of the invention is characterized, *inter alia,* by an aldehyde dehydrogenase positive phenotype. Accordingly, the cell population of the present invention can be isolated from heart tissue using methods which include the detection of aldehyde dehydrogenase (ALDH) activity.

Methods of detecting aldehyde dehydrogenase activity are known in the art. In particular embodiments of the invention the (elevated) aldehyde dehydrogenase activity is detected at the biochemical level by contacting the cells with a substrate of aldehyde dehydrogenase and detecting the product of the aldehyde dehydrogenase of the cells. This can be achieved e.g. by using a substrate, optionally a detectable susbstrate, which is converted by aldehyde dehydrogenase to a product, which is optionally a detectable product whereby either one or both of the substrate and product are detected. In particular embodiments use is made of a labeled substrate and/or product. Suitable labels include but are not limited to luminescent labels, fluorescent labels, magnetic labels, etc.. In particular embodiments the label is a fluorescent label. In more particular embodiments the substrate is a BODIPY-aminoacetaldehyde (BAAA), which is converted to a negatively charged reaction product BODiPY-aminoacetate, which can be detected as by green fluorescence channel (520-540 nm).

In particular embodiments, where detection of ALDH is based on detection of the product, the product can be prevented from leaving the cells by addition of an inhibitor of the ATP binding cassette (ABC) transporter system. Inhibitors of the ABC transporter system are known in the art (e.g., review by Cnubben et al. Informa Pharm Science 2005, Vol. 1, No. 2, Pages 219-232).

Additionally or alternatively, the cell population of the present invention can be identified and/or selected based on one or more other characterizing features, such as the expression of typical cellular markers. More particularly, the freshly isolated CASC cardiac stem cell population of the invention can be identified and/or isolated based on a typical expression pattern of one or more of the following markers: CD34, CD45, CD29, CD55, CD73 and CD117 (c-kit). Methods for determining cellular markers and/or selecting cells based on cellular markers are known in the art. Typically such detection and/or identification methods include labeled antibodies directed against the cellular marker of interest. Antibody types suitable for such uses can be, but are not limited to, monoclonal, polyclonal, single chain or recombinant antibodies. To detect binding, antibodies are typically bound either directly to a label or to a ligand, magnetic bead or enzyme. Examples of ligands include but are not limited to biotin, avidin, and streptavidin. Typically enzymes include but are not limited to luciferase, peroxidase and beta-galactosidase. Suitable ligands, beads or enzymes can be selected by a person of ordinary skill in the art. Typically for separation/isolation cells are either positively or negatively selected with labeled antibodies, such as fluorescently labeled antibodies are used in flow cytometry or antibodies are linked to magnetic beads for sorting in a magnetic apparatus or antibodies are linked to a ligand and separated on a column binding to said ligand. Other suitable known techniques involve antibodies attached to a solid matrix, e.g. plate elutriation.

According to particular embodiments of the invention, the CASCs are isolated from heart tissue based on methods which include the detection of CD34 expression. Methods for isolating CD34+ cells are known in the art and include but are not limited to sorting using magnetic beads (e.g. using the CliniMACS® system in combination with the CliniMACS CD34 Reagent).

According to further particular embodiments, the invention provides methods for obtaining a population of cardiac stem cells from isolated heart tissue, which cells are **characterized in that** they are suitable for therapeutic use, which method comprises selecting cells which are CD34 positive and/or cells which are c-kit(-) from said tissue and further comprises selecting cells which are aldehyde dehydrogenase positive and CD45 negative.

Typically methods for identifying the CASC population of the invention comprise the steps of contacting the relevant antibodies to the cellular markers with a cell population and detecting the formation of an antigen/antibody complex. Examples of such detection methods include but are not limited to radio-immuno assay (RIA), enzyme-linked immunosorbent assay (ELISA), immuno-fluorescence assay, etc...

More particularly methods for isolating/selecting the CASC population of the invention comprise the steps of contacting a sample comprising CASCs according to the invention with one or more antibodies capable of detecting the relevant cellular markers, allowing antigen-antibody complex formation and collecting the antigen-antibody complex (or, in the case of a negative marker, those cells for which there is no antigen/antibody complex formation).

Additionally or alternatively the cell population of the invention can be further characterized by the ability to differentiate into cardiomyocytes. Methods of differentiating stem cells into differentiated cells *in vitro* are known in the art. More particularly in order to determine the ability to form cardiac cells, the stem cells are typically placed in a cardiomyogenic environment. For instance, cocultivation of the CASC cardiac stem cells of the invention having the ability to differentiate into cardiomyocytes with cardiac cells will induce such stem cells to differentiate into a cardiac phenotype. The ability of the CASC population of the invention to differentiate into cardiomyocytes by being placed in a cardiomyogenic environment without the need for additional differentiation factors is an important advantage in the context of their therapeutic application. More particularly, the fact that cells differentiated out of the CASC population according to the present invention have the functional characteristics of cardiomyocytes, ensures the therapeutic potential of these cells. The CASC population of the invention can be administered to the area of the heart in need of repair and will be induced by the environment to differentiate into cardiomyocytes thereby contributing directly to heart repair.

The differentiation into cardiomyocytes can be determined in different ways. Most typically, expression of genes characteristic of cardiac tissue is determined. More particularly expression of cardiac-specific genes such as α*-actinin, myosin heavy chain* and *GATA-4,* cardiac troponin T and cardiac troponin I can be determined. Methods for determining the expression of genes are known in the art. They include both mRNA and protein-based detection methods such as but not limited to RT-PCR, immunological detection of the proteins expressed (e.g. Immunofluorescence). Additionally or alternatively the differentiation into cardiomyocytes can be determined based on morphological characterization of the cells as cardiomyocytes using transmission electron microscopy (TEM). More particularly, the presence of a sarcomeric organization in cells is typical for cardiomyocytes. Additionally or alternatively the differentiation of a cell population into cardiomyocytes can be determined by a functional characterization of the cells, e.g. using electrophysiological measurements. The cardiomyocytes obtainable by differentiation from the CASC cells of the present invention, display cardiac-specific cell markers, display a sarcomeric organization typical of cardiomyocyte morphology and exhibit typical cardiac action potentials and the three essential cardiac currents of cardiomyocytes.

The CASC population of the present invention can be isolated from heart tissue. It is envisaged that tissue from different parts of the heart can be used for the generation of the cell population of the invention, such as left and right atrium, left and right ventricle, tricuspid valve, pulmonary valve, mitral valve, aortic valve. Right atrium includes atrial appendage, fossa ovalis, limbus of fossa ovalis, crista terminalis, valve of the inferior vena cava, valve of the coronary sinus. Left atrium includes left atrial appendage. In particular embodiments the cell population of the invention is obtained from right atrial appendages.

It is envisaged that the CASC population of the present invention can be isolated from mammal heart tissue. In particular embodiments the tissue is human heart tissue. However, isolation of CASCs from heart of primates, livestock and domestic animals in the context of therapy is also envisaged.

As detailed above, the CASC cardiac stem cell population of the invention is characterized by a number of features which differentiate them from other cells present in heart tissue. Accordingly, the CASC cardiac stem cell population can be isolated from heart tissue using a variety of methods based on these features, including, but not limited to those described and/or exemplified herein. Thus a further aspect of the invention provides methods for isolating the CASC population of the invention or compositions comprising the CASC population of the invention from heart tissue, which are based on detecting one or more of the features of the cell population described herein.

Methods for identifying and selecting cells are known in the art and include methods for detection and selection based on cellular markers such as those described hereinabove. Such methods may involve selection based on one marker or based on two or more markers whereby selection based on multiple markers is performed simultaneously or consecutively. Such methods include methods which combine identification and/or selection based on cellular markers with morphological or other characteristics of the cells.

In particular embodiments, such methods comprise the step of positively selecting the cells based on detection of ALDH. While still somewhat heterogeneous, the ALDH+ population obtainable from heart contains about 80% cells which are CD34(+) and CD45(-), corresponding to the CASC CSCs of the present invention. This percentage is sufficiently high to make this population suitable for further expansion and/or therapeutic use.

In further particular embodiments such methods further comprise one or more steps based on expression of cell surface markers such as CD34, CD54, CD117 (c-kit). More particularly, the methods comprise the step of positively selecting the cells based on ALDH and positively selecting the cells based on CD34 expression. More particularly, methods may comprise selection on ALDH, CD34 and CD45 expression.

In further particular embodiments, such methods comprise the step of positively selecting the cells based on CD34 expression. The CD34+ population obtainable from heart tissue is ALDH+ and CD45- and essentially corresponds to the CASC population of the present invention.

According to further embodiments the methods of the invention comprise the step of negatively selecting the cells based on CD117 (c-kit) expression. More particularly such methods may additionally involve positively selecting the cells based on CD34 expression.

Further embodiments of the methods of the invention include methods comprising different combinations of steps selected from a number of steps including but not limited to (i) ALDH activity, (ii) expression of CD34, (iii) expression of CD45, (iv) expression of CD73, (v) expression of CD90, (vi) expression of CD105, (vii) expression of CD117, (viii) morphological characterization, (ix) expression of markers of the cardiac lineage, (x) ability of cells to differentiate into cardiomyocytes, (xi) lack of ability to differentiate into osteogenic or adipogenic lineage... etc. Suitable combinations of steps can be identified by the skilled person based on the disclosure herein and include but are not limited to combinations of two, three, four, five, six or more selection steps. More particularly methods are envisaged which combine any one of steps (i), (ii) or (vii) as a first selection step from heart tissue with one or more other steps, such as, but not limited to step (iii).

A further aspect of the present invention provides devices, tools and kits for identifying, detecting and/or isolating the novel cell population of the invention.

Suitable devices for identification and/or selection of the cells are known in the art and include for example cell sorters such as but not limited to flow cytometers such as the BD influx® cell sorter, magnetic beads such as CIiniMACS® and other kits such as Aldefluor® and Aldesort®. However other devices and tools can be developed based on similar principles. These can be adjusted or specifically developed for the identification of the CASCs or CASC population of the present invention.

Accordingly, the kits, tools and devices envisaged herein are combinations of tools suitable for identifying the CASC cardiac stem cells of the invention, more particularly tools suitable for determining the expression of suitable markers optionally combined with other features of the cells of the invention described herein.

The CASC CSC population of the invention typically refers to a population of freshly isolated cells from heart tissue. This cell population can be expanded *in vitro* while maintaining the ability to differentiate into cardiomyocytes. This population is referred to herein as a population of expanded cardiac stem cells of the invention or "expanded CASC" population and the cells are referred to as "expanded CASCs". Suitable conditions for expansion ex *vivo* or *in vitro* are known to the skilled person and include but are not limited to those described in the examples section herein. In particular embodiments, methods for expansion of CASCs of the invention involve cultivating the cells in medium comprising factors such as one or more growth factors, valproic acid, and/or the introduction of specific genes eg. HoxB4. In particular embodiments methods for expansion of the CASCs of the invention involve cultivation in the presence of platelet lysate.

The ability of *in vitro* expansion is important for therapeutic applications where a larger number of cells may be required. Accordingly, a further aspect of the invention relates to an expanded population of CASCs of the invention. Upon expansion, a number of features of the population change. More particularly, expression of CD34 is lost. However, the cells maintain their ALDH activity. Moreover, clonogenicity of the cells is also maintained. The expanded CASC cardiac stem cell population of the invention is capable of differentiating into cardiomyocytes and is thus equally suitable for therapeutic applications. Accordingly, the present invention provides methods for generating an expanded population of CASCs, which method comprises isolating CASCs as described herein and expanding them such as to obtain an expanded population of CASCs.

The CASCs, expanded CASCs and compositions comprising the CASCs of the present invention have a number of therapeutic applications. More particularly they can be used in the treatment of degenerative heart diseases and heart injury repair.

The CASC cardiac stem cells of the present invention are envisaged for use in cardiac muscle regeneration in a number of indications, including, but not limited to: (i) ischemic heart implantations, (ii) therapy for congestive heart failure patients, (iii) prevention of further disease for patients undergoing coronary artery bypass graft, (iv) conductive tissue regeneration, (v) vessel smooth muscle regeneration and (vi) valve regeneration. Thus the CASCs can also be used to integrate with tissue of a replacement heart valve to be placed into a recipient. The CASCs repopulate the valve tissue, enabling proper valve function.

Typical diseases and injuries which are susceptible to treatment by the administration of cells capable of developing into cardiomyocytes include but are not limited to congestive heart failure e.g. ischemic cardiac insults such as myocardial infarctions. Accordingly, the invention provides methods of treatment of heart injury comprising, administering the cell population of the invention to a patient in need thereof.

Accordingly, one aspect of the present invention provides the CASC cardiac stem cell population and/or expanded CASCs or composition comprising CASCs or expanded CASCs of the invention for use as a medicament. More particularly these cells and cell populations are envisaged for use in the indications described above, more particularly for use in the treatment of myocardial infarction.

A further aspect of the invention provides methods comprising the production of a population of CASCs, expanded CASCs or composition comprising CASCs or expanded CASCs according to the invention and the manufacture of a medicament for the treatment of a heart disorder using said population.

In therapeutic applications described herein, the cardiac stem cell population of the invention is used, in order of preference, as an autologous, allogeneic or xenogeneic cell population, whereby the choice largely depends on the urgency of the need for treatment. A patient presenting an imminently life-threatening condition may be maintained on a heart/lung machine while sufficient numbers of autologous CASCs are cultured or initial treatment can be provided using other than autologous CASCs.

In particular embodiments the CASCs according to the present invention are envisaged to be used for the generation of personalized medicaments. Indeed, the cells as such as well as their number and their properties may be selected based on the requirements of the patient.

The CASC therapy of the invention can be provided by several routes of administration. In particular embodiments, intracardiac muscle injection is used, which avoids the need for an open surgical procedure. The CASCs may be introduced in an injectable liquid suspension preparation or in a biocompatible medium which is injectable in liquid form and becomes semi-solid at the site of damaged myocardium. A conventional intracardiac syringe or a controllable arthroscopic delivery device can be used so long as the needle lumen or bore is of sufficient diameter that shear forces will not damage the CASCs. The injectable liquid suspension CASC preparations can also be administered intravenously, either by continuous drip or as a bolus. During open surgical procedures, involving direct physical access to the heart, all of the described forms of CASC delivery preparations are available options.

Representative examples of a dose range are volumes of about 20 to about 2000 µl, more particularly, about 500µl of injectable suspension. The amount of cells to be injected will be determined by the size of the injury sustained by the patient. Typically, at least 1x10⁵ cells and preferably 1x10⁶ cells to 1x10⁹ or more will be administered The frequency and duration of therapy will vary depending on the degree (percentage) of tissue involvement.

Injection medium can be any pharmaceutically acceptable isotonic liquid. Examples include phosphate buffered saline (PBS), culture media such as X-vivo medium, DMEM (preferably serum-free), physiological saline or 5% dextrose in water (D5W).

The cell population of the present invention may be co-administered with suitable growth factors, such as cytokines. However, the ability of the cells of the present invention to differentiate into cardiomyocytes in the absence of growth factors makes it possible to make use of the (expanded) c-kit(-) Cardiac Stem Cell population as such, without the requirement of additional growth factors for differentiation. However, growth factors may be used for expansion. In particular embodiments the CASCs of the invention are administered in combination with growth factors.

The (expanded) CASC cardiac stem cells or population of the present invention can be used as such or in combination with other cell types. For instance it is envisaged that combinations with c-kit(+) cardiac stem cells, combinations with differentiated cardiomyocytes, combinations with HSCs or combinations with other cell types can be advantageous for particular applications. In addition, as detailed herein, populations comprising at least about 70%, more particularly at least about 80% or more CASCs according to the invention (such as total ALDH+ populations isolated from heart) have also been found to be suitable for therapeutic use.

Accordingly, the present invention provides for compositions comprising CASC cardiac stem cells of the invention and/or expanded CASC cardiac stem cells optionally combined with other cell types such as but not limited to ALDH+/CD34(-) cells isolated from heart, c-kit(+) cardiac stem cells or HSCs or combinations thereof. Such compositions are provided for use in the therapeutic applications described herein. More particularly such compositions may comprise at least 20%, more particularly at least 30%, 40%, 50%, or 60%, most particularly at least 70% CASCs or expanded CASCs of the invention. In particular embodiments the compositions comprise about 80% CASCs or expanded CASCs according to the invention.

Additionally or alternatively, the (expanded) CASC cardiac stem cells or population of the present invention can be used in combination with growth factors and/or cytokines. A non-limiting list of suitable growth factors and cytokines includes, but is not limited to fibroblast growth factor (FGF), insulin-dependent growth factor (IGF), Flk-2/Flt-3 ligand (FL), stem cell factor (SCF), thrombopoietin (TPO), granulocyte-colony stimulating factor (G-CSF), megakaryocyte growth and development factor (MGDF), neuregulin-1 (NRG1β), interleukin-3 (IL-3), and IL-6. Accordingly, the present invention provides for compositions comprising CASC cardiac stem cells of the invention and/or expanded CASC cardiac stem cells optionally combined with one or more growth factors and/or cytokines.

The cells, cell population or compositions of the invention can be administered as such or provided on a scaffold. This may be in the form of one or more layers of a flexible, solid matrix that is implanted in its final form such as impregnated fibrous matrices. The matrix holds the CASCs in place at the site of injury. Examples of suitable matrices are known in the art and include but are not limited to collagen, polylactic acid, polyglycolic acid, polyurethane, Dacron, matrigel etc. as well as decellularized human (homologous) tissue. The scaffold may be biodegradable or permanent. Typically permanent scaffolds are used to replace functional parts of the heart, e.g. valves or vessels.

It is furthermore envisaged that the cells of the present invention are suitable for transplantation and gene therapy applications.

Accordingly a further aspect of the invention relates to scaffolds seeded with the Cardiac Stem Cells of the present invention and methods for seeding scaffolds *in vitro* which make use of the CASCs of the present invention. Typically the CASCs of the invention are cultivated in a bioreactor as described herein in the presence of the scaffold material. Materials suitable for use as scaffolds are known to the skilled person.

A further aspect of the invention provides the use of the CASC cardiac stem cell population and/or expanded CASCs for use in the generation of functional cardiac cells. Indeed, the ability of CASCs to differentiate into functional myocytes, including in vitro, and in addition, the ability to expand the CASCs maintaining this ability to differentiate into functional myocytes has been demonstrated. Thus, the CASCs according to the invention are an important source of cardial myocytes. Accordingly, the invention provides methods for the generation of cardiomyocytes, which involve the cultivation and differentiation of the CASCs according to the present invention. In particular embodiments, these methods involve the monocultivation of CASCs and their differentiation into functional cardiomyocytes. Alternatively, co-cultivation of CASCs is also envisaged. The functional myocytes obtainable by the methods of the present invention are suitable for therapeutic use in the treatment of heart conditions such as but not limited to congestive heart failure e.g. ischemic cardiac insults such as myocardial infarctions. Accordingly, the invention provides methods of treatment or prevention of a heart injury comprising, administering the cardiomyocyte population obtainable from the CASCs of the present invention to a patient in need thereof.

The specifications described above (e.g. dosage, source, cocultivation, optional use of a scaffold) in connection with the therapeutic use of CASCs of the invention are similarly applicable to the therapeutic use of cardiomyocytes obtainable from cultivation of the CASCs.

### BRIEF DESCRIPTION OF THE FIGURES

The following examples are intended to illustrate the invention without implying any limitation of the invention to the specific embodiments described therein. These examples are illustrated, in a non-limiting way, by the following Figures:

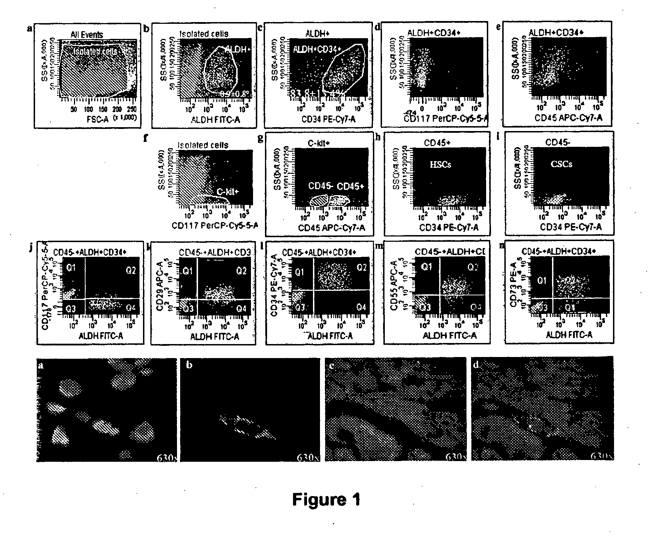

- **Figure 1:**: **Flow cytometric analysis and immunofluorescent characterization of freshly isolated CASCs according to an embodiment of the invention** A: Forward scatter/side scatter (FSC/SSC) of the total isolated cell population (a). 0.9±0.8% of the total cell population is ALDH+ (b). Within this population 83.8±13.4% of the cells express CD34 (c). These ALDH+CD34+ cells do not express CD117 (d) or CD45 (e) (CASCs). Flow cytometric analysis of the c-kit+ cells within the total population (f) shows both c-kit+CD45+ and c-kit+CD45- cells (g). All c-kit+CD45+ cells do also express CD34 (HSCs) (h), all c-kit+CD45- cells are also negative for CD34 (CSCs) (i). Flow cytometric comparison between CASCs and CSCs show that these cells differ in ALDH activity (j) and expression of CD117 (j), CD29 (k), CD34 (I), CD55 (m) and CD73 (n). B a-d (overlay): Immunofluorescent staining of CASCs present in paraffine embedded heart tissue slices. Three cells expressing CD34 (green) (b,d). Expression of cTnT (red) (c,d) could be detected in surrounding adult cardiomyocytes. Nuclei are stained with DAPI (a,d)

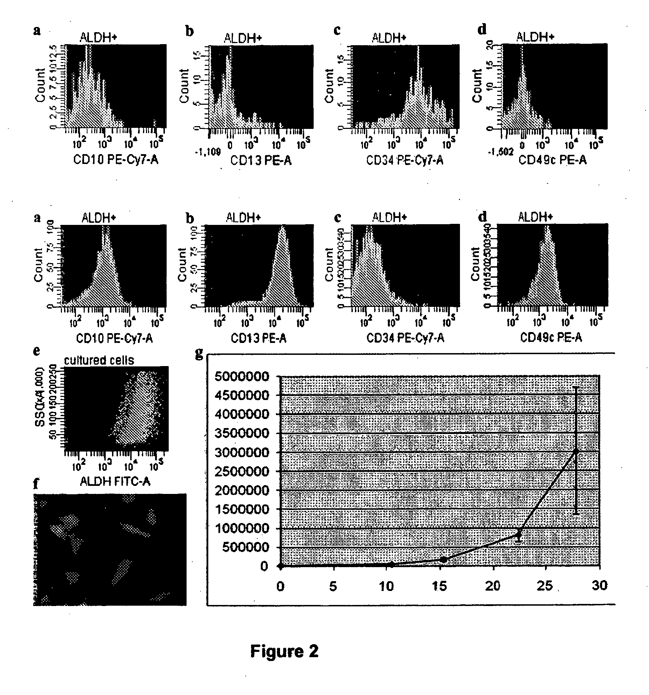

- **Figure 2:**: **Flow cytometric comparison between freshly isolated and *ex vivo* expanded CASCs according to an embodiment of the invention** A: Freshly isolated CASCs do express CD34 (c) but are negative for CD10 (a), CD13 (b) and CD49c (d). B: *Ex vivo* expanded CASCs lose their expression of CD34 (c). Expression of CD10 (a), CD13 (b) and CD49c (d) is induced by culturing these cells. However these cells still possess ALDH activity as shown by flow cytometric (e) and fluorescent microscopic (f) analysis. A representative growth curve of freshly isolated CASCs (g).

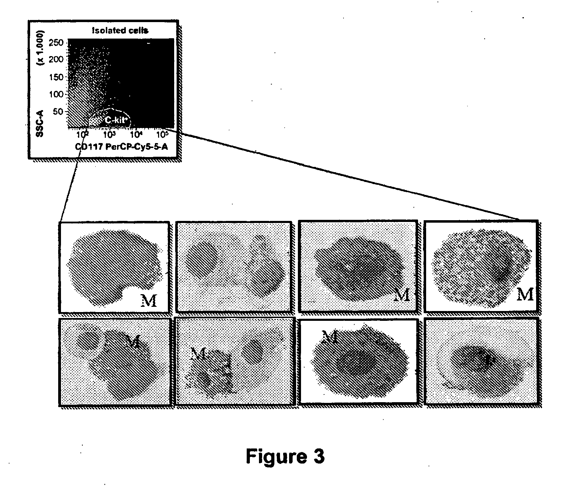

- **Figure 3:**: **Flow sorting of C-kit + cardiac stem cells from heart tissue** C-kit + cells were flow sorted from heart tissue and cytospins were made on microscope slides. Cells were stained with May Grünwald Giemsa staining. Mast cells are indicated with M.

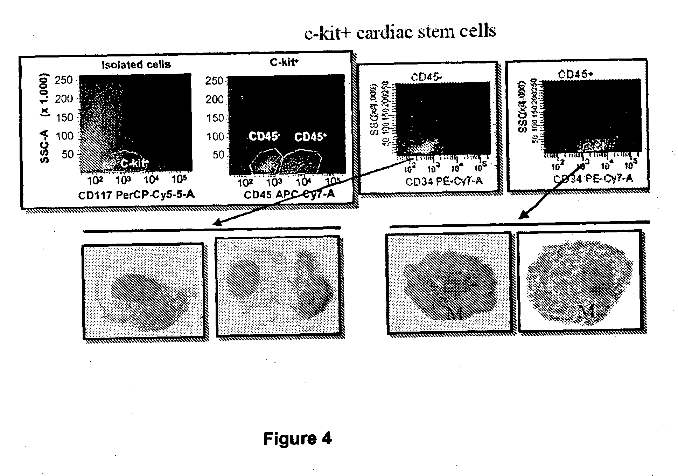

- **Figure 4:**: **Detection of CD45 expression in c-kit-+ cell population** Within the population of c-kit + cells a CD45+ and a CD45- cell population can be distinguished. The CD45+ population is considered as corresponding to a mast cell population (known to be CD45+), whereas the CD45- population is suggested to correspond to a c-kit-+/CD45- cardiac stem cell population.

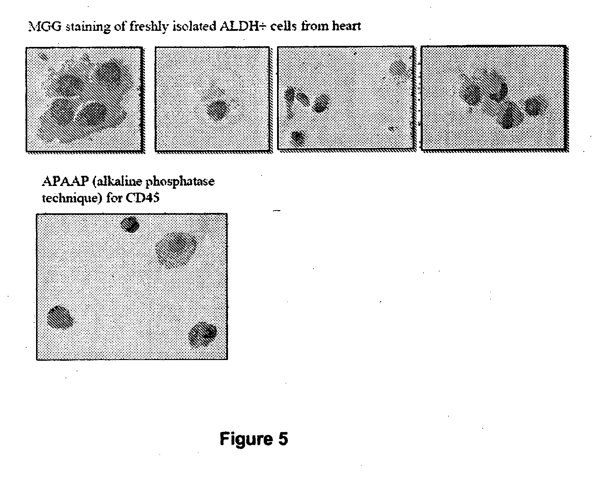

- **Figure 5:**: **Staining of freshly isolated ALDH+ cells from heart with MGG and with APAAP (alkaline phosphatase technique) for CD45** Cells were found to be medium sized with a round to oval nucleus. Nucleus was either eccentric or in the middle of the cell. Cells showed a small nuclear to cytoplasm ratio. Almost no vacuolisation was present. An immunohistochemical staining against CD45 confirmed the flow data that the cells were CD45 negative.

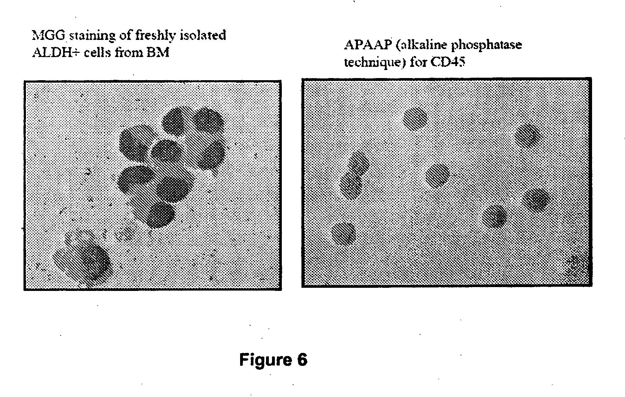

- **Figure 6:**: **Comparison of the phenotype of the ALDH+ population from heart to the phenotype of ALDH+ population isolated from bone marrow and with APAAP (alkaline phosphatase technique) for CD45** ALDH+ population isolated from bone marrow contain small cells with a nucleus which is mostly eccentric and sometimes containing nucleoli. Cells have a high nuclear to cytoplasm ratio. No vacuolisation is present. Cytoplasm is highly basophilic. A immunohistochemical staining against CD45 showed clearly that the cells are CD45 positive. Sometimes bigger cells are present containing more cytoplasm which sometimes contains vacuoles

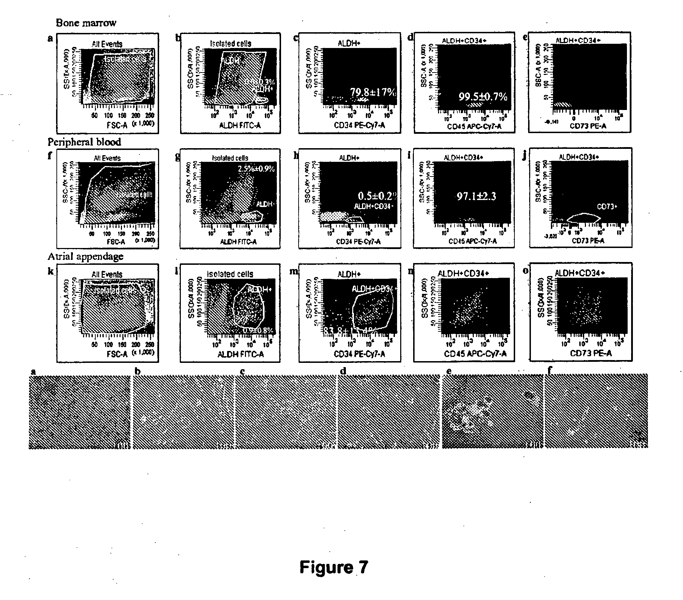

- **Figure 7:**: **Flow cytometric and functional comparison between ALDH+ cells derived from bone marrow, peripheral blood and heart tissue** A (a-e): Flow cytometric analysis of the antigen expression profile of bone marrow-derived ALDH+ cells. Within the total population (a) 0.9±0.3% of the cells are ALDH+ (b). 79.8±1.7% of these cells express CD34 (c). This double positive population is positive for CD45 (d) but does not express CD73 (e). A (f-j): Flow cytometric analysis of the antigen expression profile of peripheral blood-derived ALDH+ cells. 2.5±0.9% of the cell population (f) is ALDH+ (g). Only 0.5±0.2% of these ALDH+ cells express CD34 (h). This ALDH/CD34 double positive population expresses CD45 (i) but is negative for CD73 (j). A (k-o): Flow cytometric analysis of freshly isolated ALDH+ cells derived from heart tissue. 0.9±0.8% within the total population (k) are ALDH+ (I). ALDH+ cells do express CD34 (83.8±13.4%) (m). These cells are CD45 (n) negative but do express CD73 (o). B (a-f). Functional comparison between bone-marrow derived (a,c,e) and heart tissue derived (b,d,f) ALDH+ cells. ALDH+ cells derived from bone marrow samples contain mesenchymal stem cells that can be expanded and were able to differentiate into adipocytes (oil-red-o staining) (a) and osteoytes (Alizarin red staining) (c) When fresh ALDH+ isolated cells from BM were seeded in methocoult medium they were able to form burst-forming unit, erythrocyte (BFU- E), colony-forming unit, granulocyte-erythrocyte-monocyte-megakaryocyte (CFU- GEMM) and colony-forming unit, granulocyte-macrophage/monocyte (CFU-GM) (e) while ALDH+ cells derived from heart tissue does not contain these MSC or a HSC functional phenotype (b,d,f).

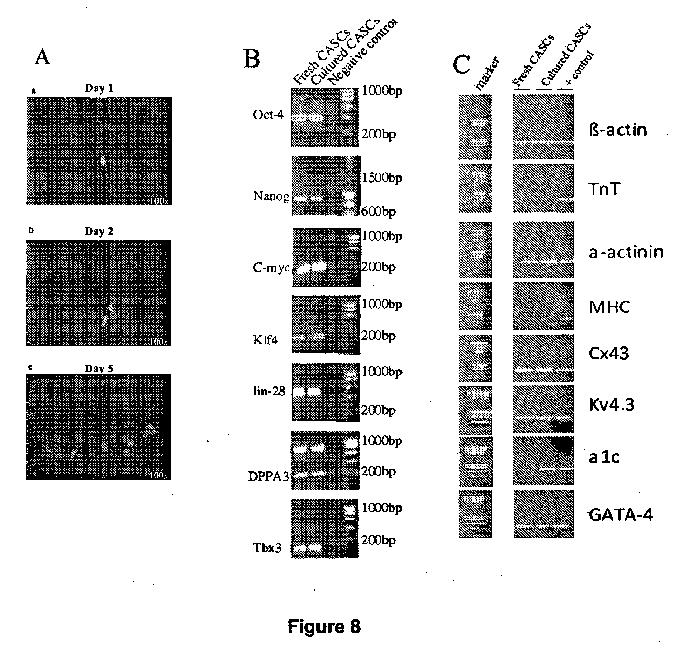

- **Figure 8:**: **Characterization of stemness of CASCs** A (a-c): After culture CASCs were still clonogenic. A total of 3456 cells were isolated and 535 clones were generated. This resulted in a clonogenicity of 15,5% for these CASCs. B: RT-PCR on RNA isolated from freshly isolated and ex *vivo* expanded CASCs to determine expression of several pluripotency associated genes. Both freshly isolated and ex *vivo* expanded CASCs expressed *Oct-4, DPPA-3, lin-28, c-myc, K*/*f-4* and *Tbx-3.* C: RT-PCR on RNA isolated from freshly isolated and ex *vivo* expanded CASCs to determine the cardiac gene expression profile of these cells. Human cardiomyocytes were used as positive control. Expression for β*-actin,* α*-actinin, Connexin43* (Cx43), *Kv4.3* and *GATA-4* could be detected in freshly isolated CASCs while *a1c* expression was induced by ex *vivo* expansion. No expression of *troponin T* (*TnT*) or *myosin heavy chain* (*MHC*) could be observed.

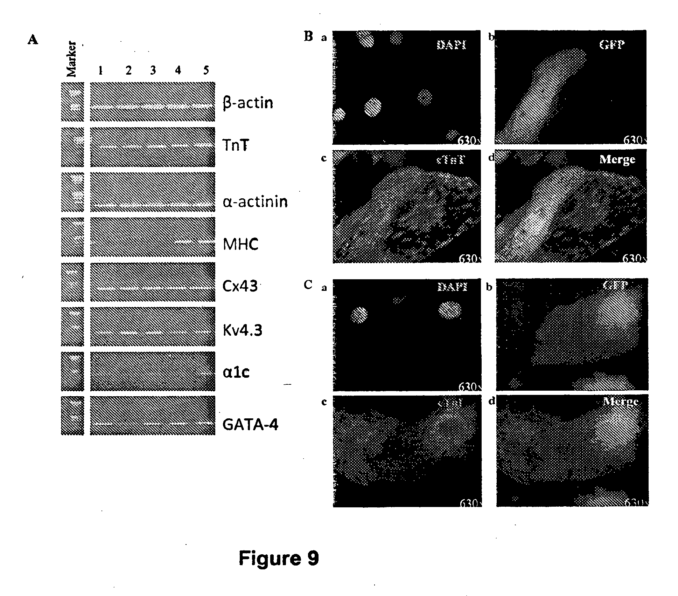

- **Figure 9:**: **Cardiomyogenic differentiation potential of *ex vivo* expanded CASCs assessed by RT-PCR and immunofluorescent staining** A: lane 1: mono-cultured CASCs; lane 2: mono-cultured CASCs in normoxic conditioned medium; lane 3: mono-cultured CASCs in hypoxic conditioned medium; lane 4: co-cultured CASCs; lane 5: human cardiomyocytes (+ control). Analysis of cardiac gene expression in mono and co-cultured CASCs. In all conditions expression of β*-actin, α-actinin, troponin T* (*TnT*), *connexin43* (Cx43), *Kv4.3, a1c* and *GATA-4* could be detected. Expression of *myosin heavy chain* (MHC) could only be detected in co-cultured CASCs. Human cardiomyocytes were used as positive control. B-C: Immunofluorescent staining for both contractile proteins cardiac troponin T (cTnT) and troponin I (cTnl) on GFP expressing CASCs after 1 week of co- culture with NRCMs. B shows positive staining for the cardiomyocyte specific protein cTnT (c,d) in GFP+ CASCs (b,d) and NRCMs (c,d). Nuclei were stained with DAPI (a,d). C shows the expression of the cardiomyocyte specific protein cTnl (e,d) in GFP+ CASCs (b,d) and NRCMs (c,d). Nuclei were stained with DAPI (a). GFP-positive cTnT or cTnl positive cells show clearly sarcomeric organization and are newly formed cardiomyocytes.

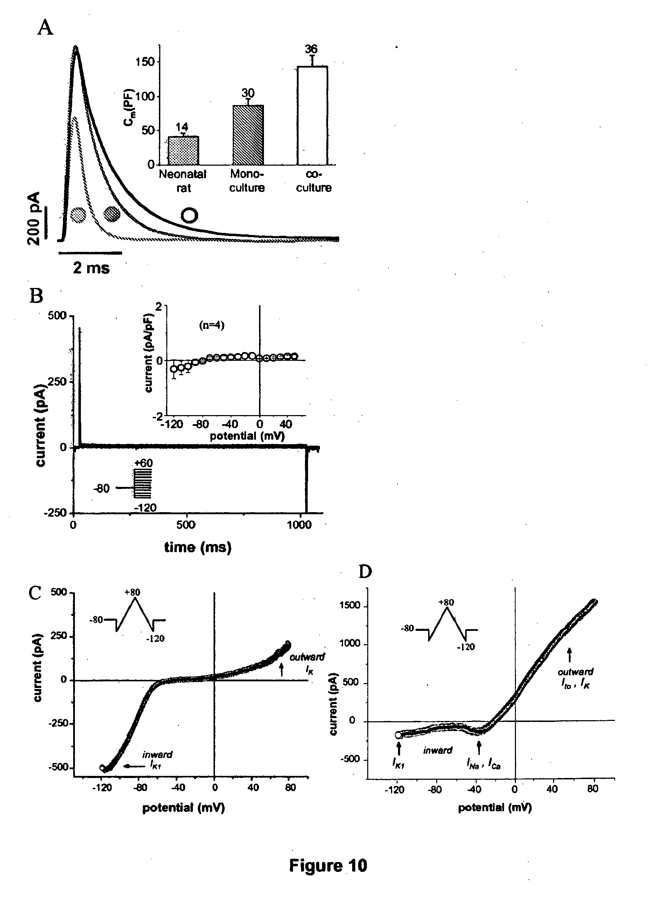

- **Figure 10:**: **Electrophysiological measurements in order to assess the functionality of CASCs** Electrophysiological measurements in order to assess the functionality of CASCs were preformed after 7 days (B) and after 8 to 10 days (C, D) of co-culture. (A) Passive electrical properties of neonatal rat cardiomyocytes (light grey), human stem cells (dark grey), and co-cultured myocytes (white). The capacity transient was recorded following a 10 mV voltage step (see Materials and Methods). In insert, mean and S.E.M. data presented. (B) Currents recorded during voltage steps, given in 10-mV increments, to potentials ranging from -120 to +70 mV. Notice no voltage-gated ion currents in CASCs after 7 days. (C and D) Appearance of inward and outward currents in CASCs after 8 to 10 days co- culture, respectively. Currents recorded during 2s ramp protocols at potentials from +80 to -120 mV.

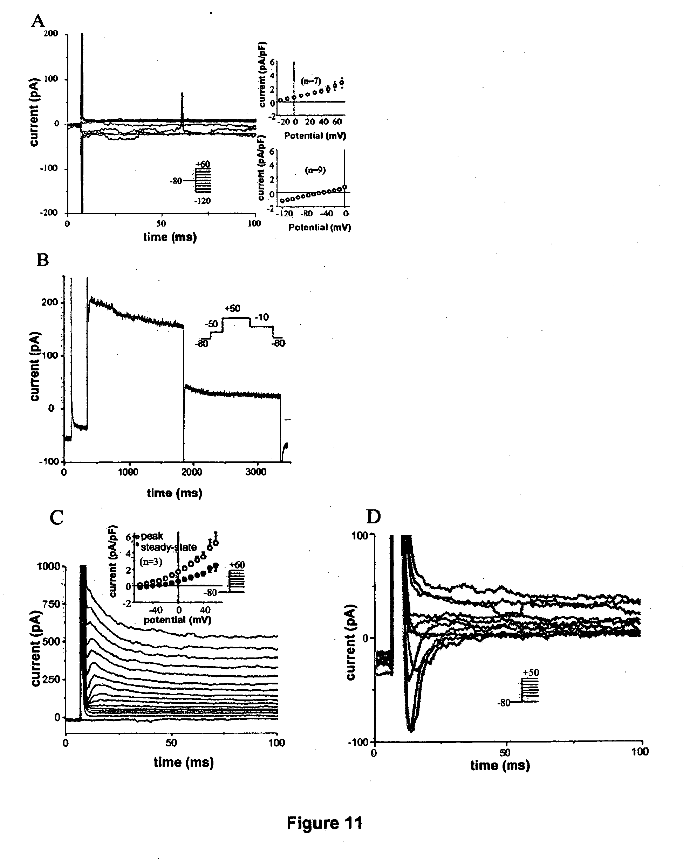

- **Figure 11:**: **Electrophysiological measurements in order to assess the functionality of ion currents** Electrophysiological measurements in order to assess the functionality of ion currents after 10 days of CASCs. (A and C). The traces of different K+ currents (IK and Ito, respectively) at various potentials recorded. (B) Depolarization activated delayed rectifier current traces (cells were depolarized to +50 mV to activate both currents (IKr and IKs) followed by a repolarization to -10 mV to deactivate IKs practically fully). (D) Voltage-gated inward current traces.

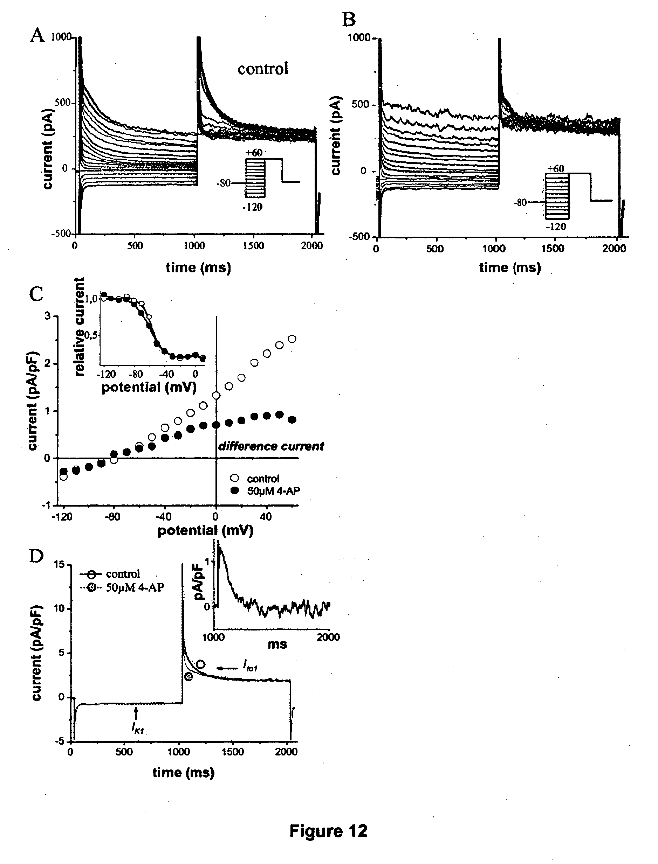

- **Figure 12:**: **Ito current** CASCs myocytes cultured for more than 10 days express an Ito. (A and B) Currents elicited by 1-s voltage steps from the holding potential (-80 mV) to +60 mV, in the absence or in the presence of 50 µM 4-AP, respectively. (C) Difference current of Ito activation vs. voltage relationship (from A and B); insert - availability (or inactivation) curve obtained at +60mV by normalizing data from A and B. (D) Ito current traces in control (white circle) and with 4-AP (grey circle), insert - the 4-AP-sensitive current, i.e., the difference between the traces in control and in the presence of the drug, is presented in insert.

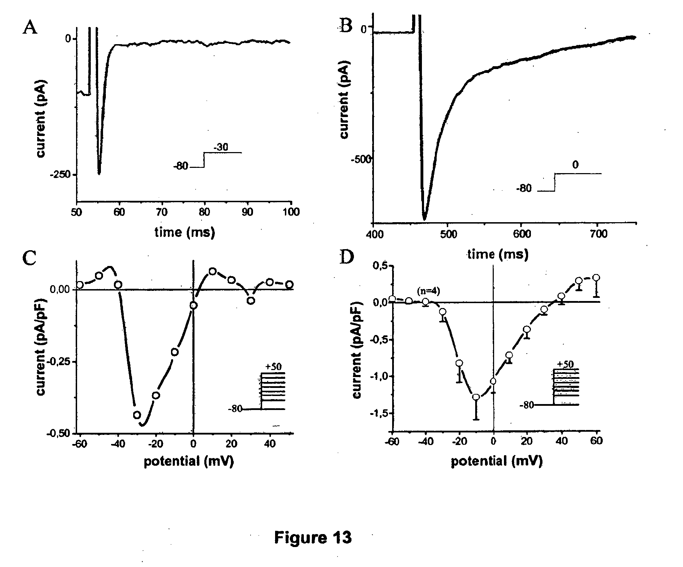

- **Figure 13:**: **Functional expression of voltage-gated ion channels in CASCs myocytes** (A and B): Na+ and Ca2+ current traces recorded at depolarising step to -30 mV and 0 mV, respectively. (C and D): Currents elicited by a family of 100-ms depolarizations from a holding potential of -80 mV to voltages ranging from -60 to +50 mV in 10-mV steps. Amplitudes of a corresponding current normalized to a cell size (current density values, pA/pF) are plotted against test voltages (mV).

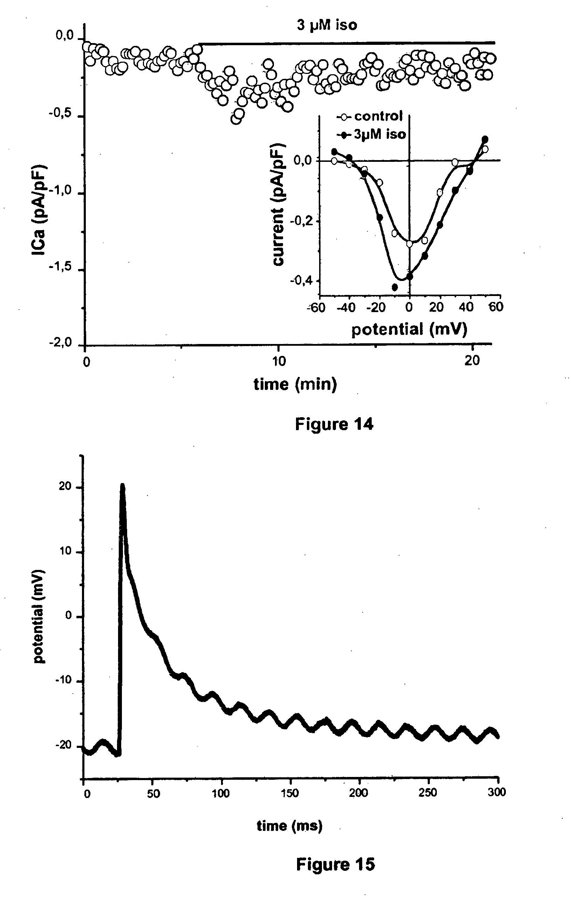

- **Figure 14:**: **Evidence for ß-adrenergic stimulation on the ICaL amplitude after 11-14 days of CASCs.**
- **Figure 15:**: **An example of slow action potential recorded in CASCs.**

### EXAMPLES

The different aspects of the invention are illustrated herein by the following non-limiting examples.

### Example 1: Isolation and expansion of a population of CASCs according to the invention from adult human heart

### a) isolation of ALDH+ cells from heart tissue

Right atrial appendages were removed during routine cardiac surgery. The heart tissue was minced and washed with Dulbecco's phosphate buffered solution (DPBS) (Lonza). The tissue fragments were dissociated by collagenase treatment (600U/ml; Invitrogen). The single cell suspension was stained with Aldefluor^{®} (Aldagen Inc) according to the manufacturers' instructions. Briefly, cells were dissolved in 1ml Aldefluor assay buffer and 5µl of activated Aldefluor^{®} was added to the cell suspension. Cells were incubated for 45 minutes at 37°C. Hereafter they were washed and the pellet was dissolved in Aldefluor assay buffer.

The average weight of the tissue was 0.8±0.3g and 0.9±0.8% of the total cell population was ALDH+ (Fig 1Ab).

### b) Flow cytometric analysis of freshly isolated and expanded CASCs

For further characterization of the freshly isolated ALDH+ cell population described above, the population was incubated for 30 minutes at 4°C with the following antibodies, CD34-Pe-Cy-7 and CD45-APC-Cy7 (both form Beckton&Dickinson). Cells were analyzed and isolated by a FACSAria^{®} (Beckton&Dickinson). Within the ALDH+ population 83.8±13.4% of the cells expressed CD34 (Fig 1Ac). ALDH+ cells expressing CD34 but negative for CD45 were isolated and collected in fetal calf serum (FCS) (Hyclone). This corresponds to the CASC population of the invention.

For expansion, the CASC population isolated as described above was centrifuged and dissolved in X-Vivo 15 medium (Lonza) supplemented with 20%FCS and 2% penicillin-streptomycin (pen-strep) (Lonza). Cells were ex *vivo* expanded in fibronectin coated culture plates (Beckton&Dickinson). The medium was changed twice a week and cells were replated with a density of 50 cells/mm² each time they reached 80-85% confluence.

No expression of c-kit (Fig 1Ad) or CD45 (Fig 1Ae) could be detected in this ALDH+CD34+ population. Flow cytometric analysis of the total cell suspension showed that CASCs and c-kit+ CSCs were phenotypically distinct populations. Analyzing the c-kit+ cells in the total cell population (Fig 1Af) revealed both CD45+ and CD45- (Fig 1Ag) cells. All CD45+c-kit+ cells expressed CD34 (Fig 1Ah) and were judged as HSCs. On the other hand, all CD45-c-kit+ cells were negative for CD34 (fig 1Ai) and were judged as CSCs (such as previously characterized by others). Not only do CASCs and c-kit+ cells differ in the expression of c-kit or ALDH activity (Fig 1Aj), they also differ in the expression of CD29 (Fig 1Ak), CD34 (Fig 1Al), CD55 (Fig 1Am) and CD73 (Fig 1An). Immunofluorescence for the detection of CD34 on paraffin embedded heart tissue slides shows that CASCs are present in the atria. Figure 1 B shows a cluster of CD34+ cells (green) surrounded by adult cardiomyocytes positive for cTnT (red).

Culturing CASCs resulted in a different antigen expression profile compared to the freshly isolated cells (Fig 2A, B). Expression of CD10 (Fig 2Aa, Ba), CD13 (Fig 2Ab, Bb) and CD49c (Fig 2Ad, Bd) was induced by the *in vitro* expansion of these CASCs. However these cultured CASCs lost their CD34 expression (Fig 2Ac, Bc) these cells kept their ALDH activity as shown by flow cytometry (Fig 2Be) and fluorescent microscopy (Fig 2Bf). Complete flow cytometric analysis of the antigen expression profile of freshly isolated and cultured CASCs is listed in Table 1. Figure 2Bg shows a representative growth curve of freshly isolated CASCs.

**Table 1: Antigen expression profile of freshly isolated CASCs and cultured CASCs**

| **Antigen** | **Fresh CASCs** | **cultured CASCS** | **Antigen** | **Fresh CASCs** | **cultured CASCs** |
|---|---|---|---|---|---|
| *CD2* | Negative | Negative | *CD55* | Positive | Positive |
| *CD3* | Negative | Negative | *CD56* | Negative | Negative |
| *CD4* | Negative | Negative | *CD59* | Negative | Negative |
| *CD5* | Negative | Negative | *CD71* | Negative | Negative |
| *CD10* | Negative | Positive | *CD73* | Positive | Positive |
| *CD11b* | Negative | Negative | *CD90* | Positive | Positive |
| *CD13* | Negative | Positive | *CD105* | Positive | Positive |
| *CD14* | Negative | Negative | *CD106* | Negative | Negative |
| *CD15* | Negative | Negative | *CD109* | Negative | Negative |
| *CD16* | Negative | Negative | *CD117* | Negative | Negative |
| *CD19* | Negative | Negative | *CD133* | Negative | Negative |
| *CD29* | Positive | Positive | *CD138* | Negative | Negative |
| *CD31* | Negative | Negative | *CD140b* | Negative | Negative |
| *CD33* | Negative | Negative | *CD144* | Negative | Negative |
| *CD34* | Positive | Negative | *CD184* | Negative | Negative |
| *CD38* | Negative | Negative | *HLA-DR* | Negative | Negative |
| *CD44* | Negative | Positive | *TIE-2* | Negative | Negative |
| *CD45* | Negative | Negative | *VEGFR-2* | Negative | Negative |
| *CD49c* | Negative | Positive | *VEGFR-3* | Negative | Negative |
| *CD50* | Negative | Negative | *ALDH* | Positive | Positive |

| | | | | | |
|---|---|---|---|---|---|
| *VEGFR-2:* Vascular endothelial growth factor receptor-2; *VEGFR-3:* Vascular endothelial growth factor receptor-3 | | | | | |

### c) Morphological characterization of the CASC population of the invention and comparison with c-kit(+) cells and ALDH+ cells from bone marrow

C-kit + cells were flow sorted from heart tissue and cytospins were made on microscope slides. Cells were stained with May Grünwald Giemsa (MGG) staining. 2 cell types were observed:
1) One celltype were typically mastcells (M). Cytoplasm contains prominent granulation. Granulation stains dark violet to purplish.
2) A second cell type are mononuclear cells with round to oval nucleus. Cells have a grey basophilic cytoplasm without granulation and sometimes vacuolisation. They have a small nucleus to cytoplasm ratio (Figure 3).

The fact that 2 cell populations were identified corresponds with the flow data described below: within the c-kit+ cells a CD45+ (yellow) and a CD45- (pink) cell population was identified. Mast cells are known to be CD45+ and probably represent the yellow CD45+ population. The other cell type corresponds with the c-kit+/CD45-cardiac stem cell population (Figure 4).

Freshly isolated ALDH+ cells from heart were stained with MGG and with APAAP (alkaline phosphatase technique) for CD45 (Figure 5).

Cells were found to be medium sized with a round to oval nucleus. Nucleus was either eccentric or in the middle of the cell. Cells showed a small nuclear to cytoplasm ratio. Almost no vacuolisation was present. An immunohistochemical staining against CD45 confirmed the flow data that the cells were CD45 negative.

The phenotype of the CASC population of the invention was compared to that of other populations, such as the ALDH+ population isolated from bone marrow (Figure 6). This latter population was found to contain small cells with a nucleus, which is mostly eccentric and sometimes containing nucleoli. Cells have a high nuclear to cytoplasm ratio. No vacuolisation is present. Cytoplasm is highly basophilic. A immunohistochemical staining against CD45 showed clearly that the cells are CD45 positive. Sometimes bigger cells are present containing more cytoplasm which sometimes contains vacuoles.

Accordingly, the CASC population of the invention is morphologically clearly different from the ALDH+ population isolated from bone marrow.

### d) Determination of antigen expression profile of non-expanded and ex vivo expanded ALDH positive cells

After the collagenase treatment and incubation with Aldefluor^{®}, cells were incubated for 30 minutes in the dark at 4°C with the antibodies listed in Table 2. The flow cytometric analysis of the ex *vivo* expanded CASCs was performed with the same set of antibodies. Cells were detached from the bottom of the culture flask with cell dissociation buffer (Invitrogen), counted and 10⁵ cells/tube were incubated with the antibodies.

**Table 2: Antibodies used for the flow cytometric characterization of CASCs**

| **Tube no.** | **FITC** | **PE** | **PerCP** | **APC** | **PE-Cy7** | **APC-Cy7** |
|---|---|---|---|---|---|---|
| **1** | CD2 | CD13 | CD19 | CD5 | CD10 | CD45 |
| **2** | CD4 | CD38 | CD3 | CD29 | - | CD45 |
| **3** | CD15 | CD44 | - | CD55 | CD34 | CD45 |
| **4** | CD16 | CD33 | CD117 | HLA-DR | CD34 | CD45 |
| **5** | CD50 | CD11b | CD117 | VEGFR3 | CD34 | CD45 |
| **6** | CD71 | CD109 | CD14 | CD90 | CD34 | CD45 |
| **7** | CD90 | CD140b | CD117 | CD133 | CD34 | CD45 |
| **8** | CD105 | CD73 | CD14 | CD184 | CD34 | CD45 |
| **9** | CD106 | CD49c | CD117 | CD90 | CD34 | CD45 |
| **10** | CD31 | CD133 | CD14 | TIE-2 | CD34 | CD45 |
| **11** | CD144 | CD133 | CD14 | VEGFR-2 | CD34 | CD45 |
| **12** | CD106 | CD49c | CD117 | CD90 | CD34 | CD45 |

FITC refers to Fluorescein isothiocyanate, PE refers to phycoerythrin-group, PerCP refers to peridinin chlorophyll a protein, APC refers to allophycocyanin, PEcy7 refers to R-phycoerythrin coupled to the cyanine dye Cy7, APC-Cy7 refers to allophycocyanin coupled to the cyanine dye Cy7:

### e) Flow cytometric analysis of bone-marrow and peripheral blood ALDH+ cells

Twenty milliliter of bone marrow was aspirated from patients' sternum before cardiac surgery and the mononuclear cell (MNC) fraction was isolated as previously described (Koninckx R 2009 Cytotherapy). For the flow cytometric analysis of peripheral blood ALDH+ cells, first a red blood cell lysis was performed by incubating the samples with ammonium chloride (NH₄Cl) in a ratio of 1:4 (blood:NH₄Cl) for 10 minutes on ice. Both samples were dissolved in 1ml Aldefluor assay buffer/5x10⁶ cells and incubated as described above. Subsequently the cells were incubated with CD34-Pe-Cy7, CD45-APC-Cy7 and CD73-PE (all from Beckton&Dickinson) for 30 minutes in the dark at 4°C.

The results of the flow cytometrical analysis of bone-marrow derived ALDH+ cells (Fig 7A a-e) and peripheral blood ALDH+ cells (Fig 7A f-j) were compared to the antigen expression profile of CASCs (Fig 7A k-o). In the bone marrow a percentage of 0.9±0.3% of the total population was ALDH+ (Fig 7Ab). Of this ALDH+ population 79.8±17% expressed CD34+ (Fig 7Ac). These double positive cells expressed CD45 (99.5±0.7%) (Fig 7Ad) but were all negative for CD73 (Fig 7Ae). In the peripheral blood 2.5±0.9% ALDH+ cells (Fig 7Ag) were detected and 0.5±0.2% of them expressed CD34 (Fig 7Ah). Of this ALDH/CD34 double positive population, 97.1±2.3% expressed CD45 (Fig 7Ai). Again all these cells were negative for CD73 (Fig 7Aj). The CASCs were ALDH+ (Fig 7Al), CD34+ (Fig 7Am), CD45- (Fig 7An) and expressed CD73 (Fig 7Ao). Based hereon it could be determined that both the ALDH+ or the CASC population isolated from heart is different from ALDH+ stem cell populations from blood or bone marrow and that the CASCs represent a novel cardiac stem cell population obtainable from heart tissue.

### f) Functional comparison between bone marrow-derived ALDH+ cells and CASCs

Bone marrow-derived MNCs were isolated and incubated as described above. ALDH+ cells were isolated by flow sorting and cultured as previously described (Koninckx R 2009 Cytotherapy) to obtain cultures of mesenchymal stem cells (MSCs). In order to control whether CASCs were functional distinct from bone marrow-derived ALDH+ MSCs, a multi-lineage differentiation assay was performed. Both cell types were forced to differentiate into adipocytes and osteocytes as previously described (Koninckx R 2009 Cytotherapy).

Hematopoietic stem cell cultures were set up by isolating ALDH+ cells from bone marrow. Thereafter both ALDH+ cells and CASCs were seeded in MethoCult^{®} GF H4434 (Stem cell Technologies). Cells were incubated for 2 weeks at 37°C and 5%CO₂ air atmosphere. After 14 days the number of burst-forming unit, erythrocyte (BFU-E), colony-forming unit, granulocyte-erythrocyte-monocyte-megakaryocyte (CFU-GEMM) and colony-forming unit, granulocyte-macrophage/monocyte (CFU-GM) were counted.

To assess whether both CASCs and ALDH+ MSCs were still able to convert Aldefluor^{®} after culture, 10⁴ cells were seeded in a 24-well plate. After 24h the medium was replaced by 1ml Aldefluor assay buffer supplemented with 5µl Aldefluor^{®} and incubated as described above. Uptake of Aldefluor^{®} was analyzed under the Axiovert 100 imaging fluorescence microscope (Zeiss, Jena, Germany).

The bone marrow-derived ALDH+ cells were able to differentiate into adipocytes (Fig 7Ba) and osteocytes (Fig 7Bc) while CASCs could not (Fig 7Bb, d). Furthermore, when freshly isolated bone marrow-derived ALDH+ cells were cultured in hematopoietic stem cell medium they formed BFU-E, CFU-GEMM, CFU-GM colonies (Fig 7Be). Freshly isolated ALDH+ cells from heart tissue were not able to grow these colonies (Fig 7Bf).

### g) Cloning assay and expression of pluripotency genes

To assess whether CASCs are indeed stem cells, the clonogenic potential of these cells was tested. GFP-labeled CASCs (obtained as detailed below) were sorted out in a 96-well plate in a density of 1cell/well with a FACSAria^{®} under stringent purity conditions. Single cell disposition was microscopically analyzed and wells with more than one cell were excluded. A total of 3456 cells were sorted and 535 clones were formed (Fig 8Aa-c) resulting in a cloning efficiency of 15,5%.

Furthermore, the expression of several pluripotency genes was analyzed. Before synthesizing the cDNA, integrity of the RNA was checked on the Agilent bio-analyser (data not shown). The freshly isolated CASCs expressed several pluripotent associated genes, like *OCT-4, DPPA3, Lin* 28, *c-myc, Klf-4* and *Tbx-3*. Furthermore, the ex *vivo* expansion of these CASCs did not influence the expression of these genes (Fig 8B). Also the cardiac gene expression of these freshly isolated and cultured CASCs was analyzed. Freshly isolated cells expressed β*-actin* (internal control) α*-actinin, Kv4.3, GATA-4* and *Connexin43* (Cx43) while α *1c* expression was induced by ex *vivo* expansion (Fig 8C). Human cardiac myocytes were used as positive control.

### Example 2: Differentiation of the CASC population of the invention into cardiomyocytes

### A. Lentiviral transfection of CASCs with GFP

In order to be able to follow the differentiation of the CASC population of the invention, a population of cells according to the invention was transfected with GFP.

CASCs were infected with a lentivirus, expressing GFP under the control of a cytomegalovirus promoter (pRRL-CMV-GFP; kindly provided by dr. Rob C. Hoeben, University Medical Center, Leiden, The Netherlands) (Carlotti Molecular therapy 2004).

### Transformation of E.coli and isolation of plasmids

Plasmids were transformed into TOP10F' competent E.coli (Invitrogen) and treated as described by the manufacturer. Plasmids were isolated out of the bacteria by using the Qiagen midi prep kit (Qiagen) according to the manufacturer's recommendations.

### Transfection of 293-T cells for the production of GFP-expressing lentiviruses

The day before transfection, 2x10³ 293-T cells/mm² were seeded and incubated overnight. The plasmids, pRRL-CMV-GFP, pMDLg-RRE, pRSV-REV and pCMV-VSVG were mixed with EZ lentifect (MellGen Laboratories nv) according to the manufacturers' recommendations. Viral supernatant was harvested after 48h, 60h and 72h and snap-shot frozen in liquid nitrogen. The titer of each viral stock was determined in 293-T cells.

### Transduction of CASCs with lentiviruses expressing GFP

For lentiviral transduction, 10⁴ CASCs were seeded in a 24-well plate. After overnight incubation the medium was changed by adding equal amounts of viral supernatants and fresh culture medium supplemented with 16µg/ml polybrene (Sigma). After 5h, fresh culture medium was added to the cells and incubated overnight. To augment the transduction efficiency cells underwent a second cycle of transduction. When passaged, transduction efficiency was analyzed by flow cytometry.

### B. Cocultivation

The transfected CASCs of the invention were then cocultured with neonatal rat cardiomyocytes, to determine whether this induces differentiation of the cells into cardiomyocytes.

### Co-cultures between CASCs and NRCMs

Primary cultures of NRCMs were derived from 3 to 4-day-old Whistar rats using the neonatal cardiomyocyte isolation system (Worthingthon) according to the manufacturer's instructions. NRCMs were seeded with a density of 7.5x10² cells/mm² After 48h, contractile cultures of NRCMs were incubated with 2µg/ml mitomycin C (Sigma-Aldrich) during 24h to prevent fibroblast overgrowth.

To induce differentiation, GFP expressing CASCs were co-cultured in a 1:3 (CASCs:NRCMs) ratio for 1 week in differentiation medium (DM; X-vivo 15 medium supplemented with 1 ng/ml transforming growth factor β1 (TGF-β1) (Sigma-Aldrich) and 2% pen-strep) and compared to co-cultures without additives. After 1 week CASCs were isolated out of the co-culture based on their green fluorescence using a FACSAria^{®}. To prevent RNA-degradation, the flow sorter was cleaned with bleach, diethyl pyrocarbonate (DEPC) (Fluka) treated solutions were used and isolated cells were directly sorted in 350µl cooled (4°C) RLT-buffer (Qiagen).

To assess whether direct contact between NRCMs and CASCs is an important trigger in myocardial differentiation, mono-cultures of CASCs were incubated in DM conditioned by culture medium obtained from NRCMs cultured under normoxic (5%CO₂ - 48h) or hypoxic (0.2%O₂ - 48h) conditions. Mono-cultures in unconditioned DM were set-up as negative control.

### C. Characterization

The co-cultivated transfected cells were then analysed both morphologically and physiologically.

### a) RNA extraction and RT-PCR

Total RNA was isolated using the RNeasy Micro kit (Qiagen). cDNA was synthesized using Superscriptlll reverse transcriptase and oligohexamers according to the manufacturer's instructions. The quality of the isolated RNA was checked by using the Agilent bio-analyzer 2100 and the RNA 6000 Pico Chip Kit (both from Agilent Technologies Inc). The protocol was performed as described by the manufacturers' recommendations.

PCR was performed with Taq polymerase (Roche) for 35 cycles consisting of 40 seconds at 95°C, 50 seconds at annealing temperature (AT) and 1 minute at 72°C, with a final extension step of 10 minutes at 72°C. Primers with AT and fragment size are listed in table 3 below.

**Table 3: RT-PCR primer sequences**

| Gene | Seq | Forward (FP) and Reverse (RP) primer | AT | Bp |
|---|---|---|---|---|
| β-actin | 1 | *FP: 5'-AGCGGGAAATCGTGCGTGACA-3'* | *56°C* | *791* |
| | 2 | *RP:5'-CCTGTAACAATGCATCTCATATTTGG-3'* | | |
| A-actinin | 3 | *FP:5'-CCTGCCTTCATGCCCTCCGA-3'* | *56°C* | *307* |
| | 4 | *RP: 5'-TGCTCCACGCGGTCCTGGTG-3'* | | |
| TnT | 5 | *FP: 5'-AGAGGTGGTGGAAGAGTACGAG-3'* | *56°C* | *406* |
| | 6 | *RP: 5'-GACGTCTCTCGATCCTGTCTTT-3'* | | |
| MHC | 7 | *FP: 5'- GAACACCAGCCTCATCAACC-3'* | *53°C* | *522* |
| | 8 | *RP: 5'-AGGTTGGTGTTGGCTTGCTC-3'* | | |
| Cx43 | 9 | *FP: 5'-CTTGGCGTGACTTCACTACTTTT-3'* | *53°C* | *490* |
| | 10 | *RP: 5'-GCATTTTCACCTTACCATGCTCT-3'* | | |
| Kv4.3 | 11 | *FP: 5'-CTTAAGACGATTGCAGGGAAGAT-3'* | *53°C* | *473* |
| | 12 | *RP: 5'-CTTCTTGTGGA TGGGTAGTTCTG-3'* | | |
| α1c | 13 | *FP: 5'-CTGGACAAGAACCAGCGACAGTGCG-3'* | *60°C* | *562* |
| | 14 | *RP: 5'-ATCACGATCAGGAGGGCCACATAGGG-3'* | | |
| GATA-4 | 15 | *FP: 5'-CCCCAATCTCGATATGTTTG-3'* | *50°C* | *396* |
| | 16 | *RP:5'-AGGAGCTGCTGGTGTCTTAG-3'* | | |
| rNkx2.5 | 17 | *FP: 5'-GCCCCCCAAGTGCTCTCCTGC -3'* | *58°C* | *768* |
| | 18 | *RP: 5'-AAGGTGGGAGTCGGTCCGGGTT-3'* | | |
| OCT-4 | 19 | *FP: 5'-TCAGCCAAACGACCATCTGCCGCT-3'* | *58°C* | *455* |
| | 20 | *RP: 5'-GAAGTGAGGGCTCCATAGCCTGG-3'* | | |
| DPPA3 | 21 | *FP: 5'-GTTACTGGGCGGAGTTCGTA-3'* | *50°C* | *167* |
| | 22 | *RP: 5'-TGAAGTGGCTTGGTGTCTTG-3'* | | |
| Lin-28 | 23 | *FP: 5'-CCTTGTTCCCAACCTCCTAAG-3'* | *50°C* | 994 |
| | 24 | *RP: 5'-CAGGTACAGGCTTTCCTACCC-3'* | | |
| c-myc | 25 | *FP: 5'-GTGCGTAAGGAAAAGTAAGG-3'* | *45°C* | 116 |
| | 26 | *RP: 5'-AAGACTCAGCCAAGGTTG-3'* | | |
| Klf-4 | 27 | *FP: 5'-CTGTTATGCACTGTGGTT-3'* | *45°C* | *201* |
| | 28 | *RP: 5'-GTATGCAAAATACAAACTCC-3'* | | |
| Tbx-3 | 29 | *FP: 5'-TTCCTACCTCACCGGGCG-3'* | *50°C* | *94* |
| | 30 | *RP: 5'-CCGTTGGGAGGCAGCGT-3'* | | |

| | | | | |
|---|---|---|---|---|
| TnT: troponin T; MHC: myosin heavy chain; Cx43: connexin43; vWF: von Willebrand factor; VEGFR-2: vascular endothelial growth factor receptor-2; rNkx2.5: rat Nkx2.5 | | | | |

### b) Immunofluorescence

Cells were fixed in 4% paraformaldehyde for 20 minutes and permeabilized with 0.3% Triton X (Sigma-Aldrich) at room temperature. Cells were overnight incubated with antibodies directed against cardiac Troponin T (cTnT) (1:500) (Abcam) or cardiac troponin I (cTnl) (1:500) (Chemicon) at 4°C followed by an incubation step with a sheep anti-rabbit rhodamine-labeled secondary antibody (1:10) (Millipore Corporation) for 1h at room temperature.

Paraffin embedded heart tissues were deparaffinized by incubating the tissues twice for 5 minutes in xylene and gradually dehydrated by a series of graded acetone concentrations. Heart slices were then overnight incubated with the first primary antibody, mouse-anti-human CD34 (1:100) (Beckton&Dickinson) at 4°C. Subsequently, slices were incubated with the first secondary antibody, goat-anti-mouse FITC (1:10) (Beckton&Dickinson) for 1h at room temperature. Hereafter, slices were incubated with the second primary antibody, cTnT (1:200) overnight at 4°C. Finally, tissue slices were incubated with a sheep-anti-rabbit rhodamine-labeled secondary antibody (1:10) (Millipore) for 1h at room temperature.

Immunofluorescence of both heart tissue slices and cell cultures were visualized with the Axioplan 2 imaging fluorescence microscope (Zeiss).

### c) Transmission Electron microscopy

Electronic microscopy is performed on monocultured and co-cultured CASCs. For electronic microscopy on co-cultures of CASCs with neonatal rat cardiomyocytes, it is important to discriminate the differentiated stem cells from rat cardiomyocytes. Therefore, before setting-up co-cultures, c-kit(-) cardiac stem cells are labelled with gold particles. Briefly, cells are incubated with 25µl ProteinA gold suspension/ ml medium for 24hrs at 37°C. Labelling efficiency is determined using the Aurion R-gent SE-LM system (Aurion) according to the manufactures instructions.

Electronic microscopy is performed in accordance with the protocols described in Greiser 2009 J Mol Cardiol and Driesen 2005 Cardiovascular Research.

### c) Electrophysiological recordings of co-cultured CASCs

GFP expressing CASCs were seeded onto contractile cardiomyocytes and co-cultured for 1 to 3 weeks prior to the recordings. Electrophysiology experiments used the whole-cell patch clamp technique (Hamill, Pflugers Arch, 1981) and were performed at room temperature with borosilicate glass patch electrodes. Patch pipettes were pulled from capillary glass (GB 200-8P; Science Products) on a DMZ-Universal Puller (Zeitz-Instrumente), which fire-polished the electrodes to a final resistance of 2-3MW when filled with internal solution. The electrodes were connected to an Axoclamp-2B amplifier (Axon Instruments), and command pulses were given and data acquired using pCLAMP software (Axon Instruments). The voltage-clamp protocols consisted of either steps (for 1s) from the holding potential of -80mV to various levels (to measure inward sodium and calcium currents) or 4s symmetrical ramps from - 120mV to +80 mV and back to -120mV, applied every 10 seconds. During the ascending limb of the ramp, the slow rate of depolarization (0.1V.s⁻¹) allowed activation and inactivation of the voltage dependent Na⁺ current. The potassium currents were measured during the descending limb of the voltage ramp, between +80mV and -120mV. Data were analyzed with Clampfit 8.2 (Axon Instruments) and Origin 6.1 (Microcal). *I_{Na}* and/or *I_{CAL}* was measured as the difference between the peak inward and the steady state current. Values are given as mean±S.E.M and were compared using ANOVA and/or two tailed *t*-test.

The extracellular solution during measurements was a Tyrode solution with the following composition ( in mM): 135 NaCl, 5.4 KCI, 0.9 MgCl₂, 1.8 CaCl₂, 0.33 NaH₂PO₄, 10 HEPES and 10 glucose (pH 7.4; titrated with NaOH). The composition of the pipette solution contained (in mM): 130 KCI or K-glutamate, 25 KCI, 1MgCl₂, 5 Na₂ATP, 1 EGTA, 0.1 Na₂GTP and 5 HEPES (pH 7.25; adjusted with KOH).

### D. Results: CASCs do differentiate into functional cardiomyocytes

After ex *vivo* expansion, CASCs were forced to differentiate down the cardiomyogenic lineage by culturing them for 1 week in the presence of NRCMs.

### a) RT-PCR expression analysis

RT-PCR analysis on a pure population of both mono and co-cultured GFP expressing CASCs showed the expression of β*-actin, TnT,* α*-actinin,* Cx43, *Kv4.3, a1c* and *GA TA-4* (Fig 9A). Expression of MHC was only detected in co-cultured CASCs. Immunofluorescence on ex *vivo* expanded (data not shown), mono (data not shown) and co-cultured CASCs revealed that cTnT (Fig 9B a-d) and cTnl (Fig 9C a-d) expression was only present in co-cultured CASCs.

### b) Transmission Electron microscopy

Analysis of differentiated cells by TEM confirmed the immunofluorescence results and revealed sarcomeric organization of the cells indicative of cardiomyocytes (data not shown).

### c) Electrophysiology

Electrophysiological measurements were performed on mono and co-cultured lentiviral transduced CASCs. Developmental changes in the total input capacitance of myocytes, an indirect measurement of membrane area, have demonstrated that the area of CASCs increases in size in co-culture relative to that of the mono-cultured CASCs surface (Fig.10,A). The cell membrane capacitance of CASCs (Fig 10, insert) in co-culture ranged from 64.9 to 314.4pF, with a mean±S.E.M. value of 142.7±16.3pF (n=36), i.e. significantly higher to that found in mono-cultured neonatal rat cardiomyocytes (41.3 ± 5.0pF, n=14) or human stem cells (86.5 ± 10.3pF, n=30). Thus, the passive electrical properties of co-cultured CASC myocytes correspond to mature human cardiac cells more closely than do those in mono-cultured CASCs. Despite the fact that co-cultured CASCs expressed TnT and Tnl in an organized fashion after 1 week, electrophysiological measurements using voltage ramps or voltage steps applied from the holding potential of -80 mV to potentials ranging from -120 through +70 mV revealed that these cells displayed almost no ion currents. Just at very negative potential an inward current was increased by a negligible margin at this time point. Mean data of 4 cells are presented in Fig 10, B (insert).

However, after 8-10 days these cells started to display both inwardly rectifying (Fig.10, C) and outward potassium currents (Fig.10, D) indicating that they were differentiating further down the cardiomyogenic lineage. Potassium currents in these experiments (Fig.10, C and D) represent the sum of all depolarization-activated outward components made up of the transient-outward (*I*ₜₒ) potassium current, delayed-rectifier (*I*_{K}, which might include rapidly activating, *I*_{Kr}, and slowly activating, *I*_{Ks}) K⁺ currents, and background *I*_{K1} channels. For quantification of functional K⁺ channels (Fig. 10, A, B and C) or other inward currents (Fig.11, D) in the CASCs cell membrane, the step protocol to different potentials was applied. When cells were voltage clamped at -80mV and stepped (for 100 ms or 1s) from -120 to +60mV, the time independent inward currents and outward time- and voltage-dependent K⁺ currents were recorded in 82.4% (14/17) of these cells.

Figure 11(A) shows a representative family of time independent sustained *I*_{K} currents measured under such experimental conditions in 7 out of 17 (41.2%) cells tested. Mean data of the current-voltage relationships (I-V curves) of outward and inward K⁺ current density (current amplitude normalized to cell capacitance, i.e. pA/pF) of such cells are presented in upper and lower inserts, respectively, of Fig.11. Because the outward *I*_{K} current might be composed of *I*_{Kr} and *I*_{Ks}, in some cells a combined electrophysiological approach was used to separate them (Fig.12B), however, no pharmacological tool was applied. Figure 11 (C and D) shows families of outward (*I*ₜₒ) and inward (*I*_{Ca}) time- and voltage-dependent current traces, respectively, recorded at potentials more positive to -40 mV. *I*ₜₒ, which in cardiac muscle is composed primarily of either Kv4.x or Kv1.4 subunits (Gutman et al., 2005; Nerbonne et al., 2000), under our experimental conditions was recorded in the minority (3 out of 17 cells, 17.6%) of CASC-myocytes examined in this study (Fig.12,C and Fig. 13). That current could be distinguished by its different current-activation kinetics and the voltage dependence of channel activation.

Figure 12A shows current traces elicited by 1-s voltage steps from the holding potential (-80 mV) to potentials between -120 and +60 mV (in 10 mV increments) following by step to +60 mV. The currents inactivated with time during the depolarizing step. Under control conditions, an initial rapid decrease was followed by a slow decay, and the total time-dependent outward current is composed of several components, such as fast inactivating component (*I*_{fast}), of a slowly inactivating component (*I*_{slow}), and a noninactivating component (*I*ₛᵤₛ). Another feature, attributing that current as *I*ₜₒ, is activation at potentials positive to -40 mV and inactivation during depolarizing steps to more positive potentials. In addition, in one cell a sensitivity to 4-AP (Fig. 12, B and D) was tested which allowed that current to be referred as *I*ₜₒ, since there was a selective suppression of *I*_{slow} by 50µM 4-AP, with no or marginal effect on *I*_{fast}. In the presence of 50 µM 4-AP the outward current was decreased in peak amplitude and decayed faster than in control, but the end-of-pulse current was practically unchanged (Fig.10, D). The 4-AP-sensitive current, i.e., the difference between the traces in control and in the presence of the drug, is presented in insert. Inward rectifier *I*_{K1} current (measured at -120 mV) was of small amplitude and not affected by drug.

Another finding, typical for cardiac cells, is the inward currents measured at potentials between -40 mV and +40 mV. Indeed, after 11 to 14 days these cells under our experimental conditions displayed a small inward current component (≈100 pA) that could be attributed to Ca²⁺-current, *I*_{CaL}, which was detectable in a small percentage of cells (23.5%, n=4 out of 17 cells), proving that the pore-forming α-subunit was present (Fig.11, D and S9, B and C). Also fast inward current, possibly *I_{Na},* was recorded in one co-cultured CASCs (Fig.13, A and C) that activated at more negative potentials and had much faster kinetics. Figure 13, A and B shows current traces of membrane currents with kinetics typical for *I*_{Na}, and *I*_{CaL} and derived peak current-voltage relationship (*I*-*V*) curves from CASCs myocytes in Fig.14, C and D. *I*_{caL} activated at voltages near -30 mV, peaked at about -10 mV or 0 mV and reversed near 40 mV.

Next, in one cell the effect of β-adrenergic stimulation on the *I*_{CaL} amplitude was tested (Fig.14).

When Iso was added to the external perfusion solution at a concentration of 3µM an increase of the *I*_{caL} was found (Fig.14). This preliminary experiment indicates for possible modulation of the electrophysiological properties by β-adrenergic receptors. This is of big importance since it demonstrates that cardiac myocytes derived from co-cultured neonatal rat and human stem cells are physiologically intact and is indicative of the functionality of hormonal receptors and possibly on the subsequent signaling cascades.

All these data are in accordance with the RT-PCR results showing the expression of α*1c.* Despite the presence of these currents and the expression of *Kv4.3,* a voltage gated potassium channel involved in the *Iₜₒ, _{fast}* repolarisation current, CASCs showed only slow action potentials (Fig 15) which are probably mediated by *Iₜₒ, _{slow}* regulated by kv1.4 (Kääb et al., 1998). The failure to generate ventricular like action potentials is also due to the fact that most cells displayed a more depolarized membrane potential (-40 - 0mV). However, these results indicate that these CASCs have the potential to differentiate into adult functional cardiomyocytes.

In conclusion, the inventors have described a new CSC population that can be isolated based on its specific ALDH activity. Phenotypically and functionally these CASCs are not mobilized bone marrow cells but actual stem cells residing in the heart. The stem cell characteristics of these cells were shown by the expression of several pluripotency associated genes and a high clonogenicity. Furthermore, these cells can be ex *vivo* expanded without losing these characteristics. When co-cultured with NRCMs they differentiate into functional cardiomyocytes as shown by the expression of cTnT and cTnl. Furthermore, the fact that these CASCs indeed differentiated into functional cardiomyocytes was also shown by our preliminary electrophysiological data on ion currents, including responsiveness to 4-AP and β-adrenergic agonist, which demonstrated the presence of *I_{Ca}, I_{Na}, Iₜₒ, I_{K}, I*_{K1} and slow action potentials. Therefore, differentiated CASCs characteristics correspond to those of fully differentiated cardiac myocytes.

### Example 3: Expansion of the CASC population

As described above, when the CASCs were cultured the antigen-expression profile of these cells changed. They lost the expression of CD34 and expression of CD10 and CD13 was induced. In spite of the fact that expression of CD34 was lost, these CASCs were still able to convert the ALDH substrate to its fluorescent product, meaning that they still possessed their ALDH activity. To further control the stemness of these cultured CASCs, clonogenicity and expression of several pluripotency associated genes was tested. The clonogenic assay demonstrated that ex *vivo* expanded CASCs still displayed a clonogenicity of 15,5%. Furthermore these cells expressed the same pluripotency associated genes, *Oct-4, DPPA-3, lin 28, c-myc, Klf-4* and *Tbx-3,* as the freshly isolated CASCs. RT-PCR of these freshly isolated and ex *vivo* expanded CASCs revealed that these cells had already some commitment towards the cardiac lineage. This can be concluded by the fact that CASCs did express α*-actinin, Cx43* and *GATA-4*. Therefore it can be stated that these CASCs are indeed endogenous cardiac stem cells. Despite the fact that these CASCs do not correspond to the standard phenotype ascribed to cardiac stem cells, these cells were able to differentiate in to functional cardiomyocytes. Expression of several cardiac specific genes like *TnT,* α*-actinin, MHC, Kv4.3,* α*1c* was induced by mono or co-culturing these CASCs. Despite the expression of *TnT* the protein itself (cTnT) could only be detected in co-cultured CASCs. Not only did co-cultured CASCs express cTnT, they also expressed the cardiac protein Tnl. Interestingly, when CASCs were mono-cultured in normoxic or hypoxic conditioned medium no expression of these two cardiac proteins could be detected. This indicated that besides soluble factors, cell-to-cell communication is a trigger for differentiation. The presence of sarcomeric structures as shown by immunofluorescence was confirmed by TEM. Mono-cultured CASCs only displayed the presence of myofibril bundles, indicating differentiation to a more myofibroblast like phenotype.

### Example 4: Therapeutic effect of the CASCs and expanded CASCs of the invention

The therapeutic efficacy of the CASCs of the present invention is first assessed in a pig animal model, in view of the high degree of similarity between the cardiovascular systems of man and pigs. The cells are first tested in the Adult Göttingen minipig model for heart failure after myocardial infarction as described by Schuleri et al. (2008). In order to identify the cells after transplantation, GFP-transfected cells, obtained as described above, are used.

Where human CASCs are used, the pigs are treated with immunosuppressive agents, so as to limit rejection of the human CASCs by the pigs. The administration of 15mg/kg cyclosporine twice a day has been shown to prevent an immune reaction against human foetal cardiomyocytes in a pig hartseizure model. In addition, flow-cytometric analysis has demonstrated that the CASCs of the present invention doe not express major histocompatibility complex II (MHC II), which ensures that only a limited rejection reaction takes place.

The inflammation is determined through histological analysis. Using hematoxylin-eosine-staining (H&E) the infiltration of macrophages and lymphocytes at the site of injection is tested.

In order to ensure that a high percentage of cells is maintained at the site of injury, the cells are injected intra-myocardially in the peri-infarctzone.

Myocardial infarction is induced under general anesthesia by way of ligation of the circumflex arterie, located on the left lateral side of the hart. This ligation is maintained for about 30 to 60 minutes, followed by reperfusion. The GFP-labeled CASCs are injected after induction of the infarct. When the region of the infarct becomes clearly visible, the GFP-labeled CASCs (n=10) are transplanted by injection with a 30-gauge needle (or a similar volume of vehicle (DPBS; n=10) intramyocardially in the peri-infarctzone of the pigs.

To determine whether the stem cell injection exerts a positive influence on the functional restoration of the hart after the infarct, the infarct size and cardial function is measured on regular intervals after transplantation. By comparing these data with the data obtained directly after induction of the myocardial infarct, the effect of the cell transplantation can be determined. Cardial contractility is determined by the measurement of the LVEF, as well as the left ventricle end-diastolic volume (LVEDV), the left ventricle end-systolic volume (LVESV), the myocardial wall-thickness and the regional perfusion, which provide an indication about cardiac pump function. These parameters are followed up by MRI, echography en computed tomografie (CT). These measurements are taken at 2 weeks and 2 months (maximal follow-up) after transplantation. After euthanasia, the harts are retrieved and prepared for histological analysis.

The efficacy of the CASCs of the present invention is further assessed in animal models for cardiac cryoinjury, which causes 55% of the left ventricular wall tissue to become scar tissue without treatment (Li et al., 1996 ; Sakai et al., 1999 , Sakai et al., 1999 ). Successful treatment will reduce the area of the scar, limit scar expansion, and improve heart function as determined by systolic, diastolic, and developed pressure. Cardiac injury can also be modeled using an embolization coil in the distal portion of the left anterior descending artery (Watanabe et al., 1998), and efficacy of treatment can be evaluated by histology and cardiac function. Cardiomyocyte preparations embodied in this invention can be used in therapy to regenerate cardiac muscle and treat insufficient cardiac function (U.S. Pat. No. 5,919,449 and WO 99/03973).

The efficacy of the CASCs is further investigated in the model of Ye J et al.. (1997)

It is confirmed that the CASCs and expanded CASCs of the invention are capable of repairing injured heart tissue.

### Example 5: expansion of CASCs for therapeutic use in humans

For most therapeutic applications, the CASCs of the invention need to be expanded in order to obtain a sufficient number of autologous or heterologous cells. Accordingly, heart tissue obtained by a biopsy was enzymatically treated to collect the cells and CASCs were selected based on ALDH or CD34 expression. Isolated CASCs were then grown ex vivo in medium containing human growth factors, such as medium from platelet lysate. Platelet lysate was obtained as follows: platelets were obtained from the Blood Transfusion Centre (e.g. from Leuven or Genk). Until processing, plasma suspended platelets were stored at room temperature with continuous agitation. Only platelets that had expired for transfusion in patients were used. Platelet concentrates were centrifuged at 4000rpm for 15 min. Supernatant was fractionated and stored at -20°C until use. CASCs were cultured in ex-vivo medium containing 10% or 20% of the platelet supernatant.

### Cited references:

Anversa P, Kajstura J. Ventricular myocytes are not terminally differentiated in the adult mammalian heart. Circ Res. 1998;83:1-14
Beltrami AP, Urbanek K, et al. Evidence that human cardiac myocytes divide after myocardial infarction. N Engl J Med. 2001;344:1750-7
Kajstura J, Leri A, Finato N, Di Loreto C, Beltrami CA, Anversa P. Myocyte proliferation in end-stage cardiac failure in humans. Proc Natl Acad Sci U S A. 1998;95:8801-5
Beltrami AP, Barlucchi L, Torella D, Baker M, Limana F, Chimenti S et al. Adult cardiac stem cells are multipotent and support myocardial regeneration. Cell. 2003;114:763-76
Hierlihy AM, Seale P, Lobe CG, Rudnicki MA, Megeney LA. The post-natal heart contains a myocardial stem cell population. FEBS Lett. 2002; 530:239-43
Bearzi C, Rota M, Hosoda T, Tillmanns J, Nascimbene A, De Angelis A et al. Human cardiac stem cells. Proc Natl Acad Sci U S A. 2007; 104:14068-73
Goumans MJ, de Boer TP, et al. TGF-beta1 induces efficient differentiation of human cardiomyocyte progenitor cells into functional cardiomyocytes in vitro. Stem Cell Res. 2007;1:138-49
Smith RR, Barile L, et al. Regenerative potential of cardiosphere-derived cells expanded from percutaneous endomyocardial biopsy specimens. Circulation. 2007;115:896-908
Laugwitz KL, Moretti A, Lam J, Gruber P, Chen Y, Woodard S et al. Postnatal isl1+ cardioblasts enter fully differentiated cardiomyocyte lineages. Nature. 2005;433:647-53
Wolf NS, Koné A, Priestley GV, Bartelmez SH. In vivo and in vitro characterization of long-term repopulating primitive hematopoietic cells isolated by sequential Hoechst 33342-rhodamine 123 FACS selection. Exp Hematol. 1993;21:614-22
Challen GA, Little MH. A side order of stem cells: the SP phenotype. Stem Cells. 2006;24:3-12 Yoon J, Choi SC, Park CY, Shim WJ, Lim DS. Cardiac side population cells exhibit endothelial differentiation potential. Exp Mol Med. 2007;39:653-62
Pfister O, Mouquet F, et al. CD31- but not CD31+ cardiac side population cells exhibit functional cardiomyogenic differentiation. Circ Res. 2005;97:52-61
Yamahara K, Fukushima S, et al. Heterogenic nature of adult cardiac side population cells. Biochem Biophys Res Commun. 2008;371:615-20
Storms RW, Trujillo AP, et al. Isolation of primitive human hematopoietic progenitors on the basis of aldehyde dehydrogenase activity. Proc Natl Acad Sci U S A. 1999;96:9118-23
Hess DA, Meyerrose TE, et al. Functional characterization of highly purified human hematopoietic repopulating cells isolated according to aldehyde dehydrogenase activity. Blood. 2004; 104: 1648-55
Gentry T, Foster S, Winstead L, Deibert E, Fiordalisi M, Balber A. Simultaneous isolation of human BM hematopoietic, endothelial and mesenchymal progenitor cells by flow sorting based on aldehyde dehydrogenase activity: implications for cell therapy. Cytotherapy. 2007;9(3):259-74.
Greiser M, Neuberger HR,et al.. Distinct contractile and molecular differences between two goat models of atrial dysfunction: AV block-induced atrial dilatation and atrial fibrillation. J Mol Cell Cardiol. 2009 Mar;46(3):385-94.
Ronald B. Driesen, et al. Partial cell fusion: A newly recognized type of communication between dedifferentiating cardiomyocytes and fibroblasts. Cardiovascular Research 2005 68(1):37-46
Li R, Jia Z-Q, Weisel R, Mickle D, Zhang J, Mohabeer M, Rao V, Ivanov J. Cardiomyocyte Transplantation Improves Heart Function Ann. Thorac. Surg. 62:654, 1996;
Sakai T, Li RK, Weisel RD, Mickle DA, Kim EJ, Tomita S, Jia ZQ, Yau TM. Autologous heart cell transplantation improves cardiac function after myocardial injury. Ann. Thorac. Surg. 8:2074, 1999,
Sakai T, Li RK, Weisel RD, Mickle DA, Jia ZQ, Tomita S, Kim EJ, Yau TM., Fetal cell transplantation: a comparison of three cell types.J. Thorac. Cardiovasc. Surg. 118:715, 1999
Watanabe E, Smith DM Jr, Delcarpio JB, Sun J, Smart FW, Van Meter CH Jr, Claycomb WC. Cardiomyocyte transplantation in a porcine myocardial infarction modelCell Transplant. 7:239, 1998 Ye J., Yang L, Sethi R, Copps J, Ramjiawan B, Summers R, Deslauriers R - A new technique of coronary artery ligation: Experimental myocardial infarction in rats in vivo with reduced mortality. Mol Cell Biochem 1997;176:227-233
Spicher A, Meinhardt A, Roehrich M-E and Vassali G. Phenotypic characterization of Murine and Human cardiac-resident progenitor cells isolated on basis of aldehyde dehydrogenase activity. Ciculation 2007, 116:II_167-II_168.
Nerbonne JM. Molecular basis of functional voltage-gated K+ channel diversity in the mammalian myocardium. J Physiol. 2000;525:285-98
Gutman GA, Chandy KG, Grissmer S, Lazdunski M, McKinnon D, Pardo LA et al. International Union of Pharmacology. LIII. Nomenclature and molecular relationships of voltage- gated potassium channels. Pharmacol Rev. 2005;57:473-508
Schuleri KH, Boyle AJ et al. The adult Göttingen Minipig as a model for chronic heart failure after myocardial infarction: focus on cardiovascular imaging and regenerative therapies. Comparative Medicine 2008, 58(6):568-579.
Hamill O, Marty A, Neher E, Sakmann B, Sigworth F. Improved patch-clamp techniques for high-resolution current recording from cells and cell-free membrane patches. Pflugers Arch. 1981;391:85-100

## Claims

1. A population of cardiac stem cells **characterized by** the following features:
- positive for aldehyde dehydrogenase activity
- expression of CD34
- absence of expression of c-kit, and
- absence of expression of CD45
or a pharmaceutical composition comprising said cardiac stem cells for use in the treatment of heart disease and/or heart injury.

2. The population of expanded cardiac stem cells obtainable by expansion of the population or the pharmaceutical composition comprising said cardiac stem cells for use in the treatment of heart disease and/or heart injury of claim 1, **characterized by** the following features:
- positive for aldehyde dehydrogenase activity
- absence of CD34 expression, and
- expression of CD10 and CD13.

3. The pharmaceutical composition for use in the treatment of heart disease and/or heart injury of claim 1 or 2 further comprising growth factors and/or cytokines.

4. The pharmaceutical composition for use in the treatment of heart disease and/or injury of any one of claims 1 to 3, which is an injectable solution.

5. The population or the pharmaceutical composition for use in the treatment of heart disease and/or injury of claims 1 to 4, wherein said cells are provided on a scaffold.

6. The population or the pharmaceutical composition for use in the treatment of heart disease and/or injury according to claim 5, wherein the scaffold is a flexible solid matrix.

7. The population or the pharmaceutical composition for use in the treatment of heart disease and/or injury according to claim 6, wherein the matrix is selected from impregnated fibrous collagen, polylactic acid, polyglycolic acid, polyurethane, Dacron, matrigel, and decellularized human (homologous) tissue.

8. Use of a population of cardiac stem cells **characterized by** the following features:
- positive for aldehyde dehydrogenase activity
- expression of CD34
- absence of expression of c-kit, and
- absence of expression of CD45
for the generation of cardiomyocytes in vitro.

9. Use according to claim 8, wherein said population of cardiac stem cells are differentiated into cardiac cells in monoculture.

## Patentansprüche

1. Population kardialer Stammzellen, die durch die folgenden Merkmale gekennzeichnet sind:
- positiv für Aldehyddehydrogenase-Aktivität
- Expression von CD34
- Abwesenheit der Expression von c-kit, und
- Abwesenheit der Expression von CD45
oder eine pharmazeutische Zusammensetzung, welche die kardialen Stammzellen umfasst, zur Verwendung in der Behandlung einer Herzerkrankung und/oder Herzverletzung.

2. Population expandierter kardialer Stammzellen, die durch Expansion der Population oder der pharmazeutischen Zusammensetzung, welche die kardialen Stammzellen zur Verwendung in der Behandlung einer Herzerkrankung und/oder Herzverletzung nach Anspruch 1 enthält, erhältlich sind, **gekennzeichnet durch** die folgenden Merkmale:
- positiv für Aldehyddehydrogenase-Aktivität
- Abwesenheit der CD34-Expression, und
- Expression von CD10 und CD13.

3. Pharmazeutische Zusammensetzung zur Verwendung in der Behandlung einer Herzerkrankung und/oder Herzverletzung nach Anspruch 1 oder 2, die ferner Wachstumsfaktoren und/oder Zytokine umfasst.

4. Pharmazeutische Zusammensetzung zur Verwendung in der Behandlung einer Herzerkrankung und/oder Herzverletzung nach einem der Ansprüche 1 bis 3, die eine injizierbare Lösung ist.

5. Population oder pharmazeutische Zusammensetzung zur Verwendung in der Behandlung einer Herzerkrankung und/oder Herzverletzung nach den Ansprüchen 1 bis 4, wobei die Zellen auf einem Trägermaterial bereitgestellt werden.

6. Population oder pharmazeutische Zusammensetzung zur Verwendung in der Behandlung einer Herzerkrankung und/oder Herzverletzung nach Anspruch 5, wobei das Trägermaterial eine elastische feste Matrix ist.

7. Population oder pharmazeutische Zusammensetzung zur Verwendung in der Behandlung einer Herzerkrankung und/oder Herzverletzung nach Anspruch 6, wobei die Matrix ausgewählt ist aus imprägniertem, faserförmigen Kollagen, Polymilchsäure, Polyglykolsäure, Polyurethan, Dacron, Matrigel und dezellularisiertem menschlichen (homologen) Gewebe.

8. Verwendung einer Population kardialer Stammzellen, die durch die folgenden Merkmale gekennzeichnet sind:
- positiv für Aldehyddehydrogenase-Aktivität
- Expression von CD34
- Abwesenheit der Expression von c-kit, und
- Abwesenheit der Expression von CD45
für die Erzeugung von Kardiomyozyten in vitro.

9. Verwendung nach Anspruch 8, wobei die Population der kardialen Stammzellen in Monokultur zu Herzzellen differenziert wird.

## Revendications

1. Population de cellules souches cardiaques **caractérisées par** les caractéristiques suivantes :
- positives pour l'activité aldéhyde déshydrogénase
- expression de CD34
- absence d'expression de c-kit, et
- absence d'expression de CD45
ou une composition pharmaceutique comprenant lesdites cellules souches cardiaques pour son utilisation dans le traitement d'une maladie cardiaque ou d'une lésion cardiaque.

2. Population de cellules souches cardiaques expansées pouvant être obtenue par expansion de la population ou la composition pharmaceutique comprenant lesdites cellules souches cardiaques pour son utilisation dans le traitement d'une maladie cardiaque et/ou d'une lésion cardiaque selon la revendication 1, lesdites cellules étant **caractérisées par** les caractéristiques suivantes :
- positives pour l'activité aldéhyde déshydrogénase
- absence d'expression de CD34, et
- expression de CD10 et CD13.

3. Composition pharmaceutique pour son utilisation dans le traitement d'une maladie cardiaque et/ou d'une lésion cardiaque selon la revendication 1 ou 2 comprenant en outre des facteurs de croissance et/ou des cytokines.

4. Composition pharmaceutique pour son utilisation dans le traitement d'une maladie et/ou d'une lésion cardiaque selon l'une quelconque des revendications 1 à 3, qui est une solution injectable.

5. Population ou composition pharmaceutique pour son utilisation dans le traitement d'une maladie et/ou d'une lésion cardiaque selon l'une quelconque des revendications 1 à 4, dans laquelle lesdites cellules sont fournies sur un échafaudage.

6. Population ou composition pharmaceutique pour son utilisation dans le traitement d'une maladie et/ou d'une lésion cardiaque selon la revendication 5, dans laquelle l'échafaudage est une matrice solide flexible.

7. Population ou composition pharmaceutique pour son utilisation dans le traitement d'une maladie et/ou d'une lésion cardiaque selon la revendication 6, dans laquelle la matrice est sélectionnée parmi le collagène fibreux imprégné, l'acide polylactique, l'acide polyglycolique, le polyuréthane, le Dacron, le matrigel, et le tissu humain (homologue) décellularisé.

8. Utilisation d'une population de cellules souches cardiaques **caractérisées par** les caractéristiques suivantes :
- positives pour l'activité aldéhyde déshydrogénase
- expression de CD34
- absence d'expression de c-kit, et
- absence d'expression de CD45
pour la génération de cardiomyocytes in vitro.

9. Utilisation selon la revendication 8, dans laquelle les cellules de ladite population de cellules souches cardiaques sont différenciées en cellules cardiaques en monoculture.
